# EUROPÄISCHE PATENTSCHRIFT

(11) **EP 1 664 317 B1**
(45) Veröffentlichungstag und Bekanntmachung des Hinweises auf die Patenterteilung: **06.03.2013**
(21) Anmeldenummer: 05726279.2
(22) Anmeldetag: 31.01.2005
(51) Int. Cl.: A61K 31/675, A61P 25/00

(54) **Verwendung von Precursor Z zur Herstellung eines Mittels zur Therapie humaner Molybdäncofaktor-Defizienz**
Use of precursor Z for the production of a means for therapy of human molybdenum cofactor deficiency
Utilisation du précurseur Z pour produire un agent servant à traiter la déficience humaine en cofacteur molybdène

(30) Priorität: 29.01.2004 DE 102004004642; 30.01.2004 DE 102004063948
(43) Veröffentlichungstag der Anmeldung: 07.06.2006
(73) Patentinhaber: Alexion Pharma International SARL, 1005 Lausanne (CH)
(72) Erfinder: SCHWARZ, Guenter, 53859 Niederkassel (DE); MENDEL, Ralf, 38542 Leitferde (DE); SANTAMARIA, José, 50939 Köln (DE); REISS, Jochen, 37127 Niemetal (DE)
(74) Vertreter: Vossius & Partner
(86) Internationale Anmeldenummer: PCT/DE2005/000142
(87) Internationale Veröffentlichungsnummer: WO 2005/073387

(56) Entgegenhaltungen:
- HAENZELMANN PETRA ET AL: "Functionality of alternative splice forms of the first enzymes involved in human molybdenum cofactor biosynthesis" JOURNAL OF BIOLOGICAL CHEMISTRY, Bd. 277, Nr. 21, 24. Mai 2002 (2002-05-24), Seiten 18303-18312, XP002337385 ISSN: 0021-9258
- WUEBBENS MARGOT M ET AL: "Structural characterization of a molybdopterin precursor" JOURNAL OF BIOLOGICAL CHEMISTRY, Bd. 268, Nr. 18, 1993, Seiten 13493-13498, XP002337260 ISSN: 0021-9258 in der Anmeldung erwähnt
- SCHWARZ GUERNTER ET AL: "Rescue of lethal molybdenum cofactor deficiency by a biosynthetic precursor from Escherichia coli" HUMAN MOLECULAR GENETICS, Bd. 13, Nr. 12, 15. Juni 2004 (2004-06-15), Seiten 1249-1255, XP002337257 ISSN: 0964-6906
- SANTAMARIA-ARAUJO JOSE ANGEL ET AL: "The tetrahydropyranopterin structure of the sulfur-free and metal-free molybdenum cofactor precursor" JOURNAL OF BIOLOGICAL CHEMISTRY, Bd. 279, Nr. 16, 16. April 2004 (2004-04-16), Seiten 15994-15999, XP002337258 ISSN: 0021-9258
- WUEBBENS MARGOT M ET AL: "Mechanistic and mutational studies of Escherichia coli molybdopterin synthase clarify the final step of molybdopterin biosynthesis." JOURNAL OF BIOLOGICAL CHEMISTRY, Bd. 278, Nr. 16, 18. April 2003 (2003-04-18), Seiten 14523-14532, XP002337259 ISSN: 0021-9258 in der Anmeldung erwähnt
- GUTZKE G.: "Der zweite Schritt der Molybdäncofaktor-Biosynthese: Molekularer Mechanismus der Konversion von PrecursorZ zu Molybdopterin" [Online] 14. Juni 2002 (2002-06-14), , XP002337437 Gefunden im Internet: URL:http://opus.tu-bs.de/opus/vollt.PDF> [gefunden am 2005-07-20] das ganze Dokument
- SAWERS R G ET AL: "TWO-DIMENSIONAL GEL ELECTROPHORETIC ANALYSIS OF ESCHERICHIA-COLI PROTEINS INFLUENCE OF VARIOUS ANAEROBIC GROWTH CONDITIONS AND THE FNR GENE PRODUCT ON CELLULAR PROTEIN COMPOSITION", ARCHIVES OF MICROBIOLOGY, vol. 149, no. 3, 1988, pages 240-244, XP008131499, ISSN: 0302-8933
- YOHANNES ELIZABETH ET AL: "pH-dependent catabolic protein expression during anaerobic growth of Escherichia coli K-12.", JOURNAL OF BACTERIOLOGY, vol. 186, no. 1, January 2004 (2004-01), pages 192-199, ISSN: 0021-9193

## Beschreibung

Die Erfindung betrifft die Verwendung von Precursor Z zur Herstellung eines Mittels zur Therapie humaner Molybdäncofaktor-Defizienz, die direkt oder indirekt auf eine veränderte Molybdäncofaktor-Synthese zurückzuführen sind.

Der Molybdäncofaktor (Moco), eine hochkonservierte, Molybdän (Mo)-koordinierende Pterin-Verbindung, ist für die Aktivität von allen Mo-Enzymen mit Ausnahme von Nitrogenase notwendig. Der Moco wird in einem einzigartigen und evolutionär altem mehrschrittigem Syntheseweg hergestellt, aus dem bisher zwei Intermediate identifiziert wurden: das Schwefel- und Metallfreie Pterinderivat Precursor Z und Molybdopterin (MPT), ein Pterin mit einer En-Dithiol-Funktion, die für die Mo-Bindung essenziell ist. Letztere Pterin-Komponente bildet vermutlich ein Pyronapterin ähnlich dem Moco, der in Kristallstrukturen von Mo-Enzymen gefunden wurde. MPT koordiniert ebenso Wolfram (W) in W-abhängigen Enzymen.

Aus HAENZELMANN PETRA ET AL: "Functionality of alternative splice forms of the first enzymes involved in human molybdenum cofactor biosynthesis" JOURNAL OF BIOLOGICAL CHEMISTRY, Bd. 277, Nr. 21, 24. Mai 2002 (2002-05-24), Seiten 18303-18312, ISSN: 0021-9258, ist bekannt, dass die rekombinante Expression der beiden "Precursor Z-synthetisierenden Proteine" MOCS1A und MOCS1B (Homologe der bakteriellen Proteine MoaA und MoaC) zurAkkumulation von Precursor Z führt.

Weiterhin ist bekannt, dass Precursor Z lediglich in solchen E.coli Stämmen nachgewiesen werden kann, die einen Defekt in der Umwandlung von Precursor Z zu Molybdopterin Precursor Z aufweisen, hier spezifisch: eine inaktive MPT-Synthase, die den besagten Umwandlungsschritt katalysiert.

Weiterhin ist aus WUEBBENS MARGOT M ET AL: "Structural characterization of a molybdopterin precursor" JOURNAL OF BIOLOGICAL CHEMISTRY, Bd. 268, Nr. 18, 1993, Seiten 13493-13498, ISSN: 0021-9258, die Reinigung von Precursor Z aus Molybdopterin-defizienten *chIN E.coli* Zellen mittels saurer Extraktion, einer HPLC Anionenaustauschsäule und einer Reverse-Phase/Anionenaustauscher-Säule bekannt, wobei die *E.coli* Zellen bei 37°C und ohne Induktor herangezogen wurden.

Ersatztherapien für Erbkrankheiten, einschliesslich Methoden zum Ersatz von Enzymen, sind häufig durch die begrenzte Verfügbarkeit des erforderlichen therapeutischen Wirkstoffes beschränkt.

Molybdän-Cofaktor-(Moco-) Defizienz ist eine pleiotrope genetische Fehlfunktion, die sich durch den Ausfall der molybdänabhängigen Enzyme Sulfit-Oxidase, Xanthin-Oxidoreduktase und Aldehyd-Oxidase aufgrund von Mutationen in Moco-Biosynthese-Genen auszeichnet. Ein Intermediat dieses Biosynthese-Weges, "Precursor Z", ist stabiler als der Cofaktor selbst und besitzt eine konservierte Struktur in allen Organismenreichen. Aus diesem Grund wurde die Substanz im Bakterium *E*. *coli* produziert, gereinigt und zur Behandlung von Precursor Z-defizienten "Knock-out"-Mäusen verwendet, die in ihrem Krankheitsbild dem der humanen Cofaktor-Defizienz entsprechen. Precursor Z-behandelte Mäuse erreichen das Erwachsenen-Stadium und sind fortpflanzungsfähig. Biochemische und morphologische Analysen sowie Untersuchungen des Verhaltens geben Grund zur Annahme, dass die beschriebene Behandlung zur Abschwächung der meisten Symptome humaner Moco-Defizienz geeignet ist.

Alle Molybdän (Mo) enthaltenden Enzyme von Menschen, Tieren, Pflanzen, Archaea und Bakterien, mit der prokaryotischen Nitrogenase als einziger Ausnahme, benötigen einen Cofaktor, der aus einem organischen Gerüst, dem so genannten Molybdopterin (MPT), und Molybdän zusammengesetzt ist^{B1}. Dieser einzigartige und "universelle" Molybdän-Cofaktor (Moco) besitzt in allen phylogenetischen Gruppen die gleiche Grundstruktur und ist in freier Form sehr instabil, insbesondere unter aeroben Bedingungen, sofern er nicht an ein Apo-Protein gebunden ist^{B2}. Der mehrstufige Biosyntheseweg, über den ein Guanosin-Derivat in aktiven Moco umgewandelt wird, ist evolutionär konserviert^{B1} und die entsprechenden, an der Moco-Synthese beteiligten Proteine verschiedener Organismen sind extrem homol og^{B3-B7}. Ein durch Mutation bedingter Defekt in der Moco-Biosynthese führt zum simultanen Ausfall der Aktivitäten aller Mo-Enzyme, einschliesslich der Sulfit-Oxidase^{B8,B9}. Humane Moco-Defizienz ist eine schwere, autosomal-rezessive genetische Störung, die klinisch von der weniger häufig auftretenden Sulfit-Oxidase Defizienz nicht zu unterscheiden ist^{B10-B12}. Obwohl einige weniger schwere Fälle bekannt sind ^{B13} weisen die meisten betroffenen Patienten neuronale Abnormitäten wie nicht behandelbare Anfälle und Fehlentwicklungen des Gehirnes auf, was auf die Toxizität von Sulfit, einen Mangel an Sulfat bzw. beides zurückzuführen ist. Bis zum heutigen Tag ist keine wirkungsvolle Therapie verfügbar, weshalb entsprechende Patienten in der Regel in früher Kindheit versterben^{B14}.

Die ersten eukaryotischen Gene der Moco-Biosynthese, die isoliert werden konnten, stammten aus der Pflanze *Arabidopsis thaliana ^{B15}.* Eine Suche nach homologen Sequenzen führte zur Identifikation des ersten humanen Gens der Moco-Biosynthese, *MOCS1^{B3}.* Die Genprodukte MOCS1A und MOCS1B, deren Expression ein kompliziertes Muster von alternativem Spleissen beinhaltet^{B16-B18}, wandeln ein Guanosin-Derivat in den schwefelfreien Precursor Z um, der bereits über die einzigartige, aus vier C-Atomen bestehende Seitenkette des MPT verfügt^{B19}. Mutationen im MOCS1-Gen wurden bei zwei Drittel der Moco-defizienten Patienten festgestellt, welche die Komplementationsgruppe A bzw. den Moco-Defizienz Typ A darstellen^{B10,B20}. In einem nachfolgenden Schritt wird Precursor Z zu MPT umgewandelt; dies geschieht durch ein Enzym, das vom MOCS2-Gen codiert und vom *MOCS3-*Genprodukt aktiviert wird^{B4}. Die Mehrzahl der Typ B-Patienten weist MOCS2-Mutationen auf^{B12,B13,B21}. Schließlich wird Mo durch das multifunktionale Protein Gephyrin auf MPT übertragen^{B5}. Bis jetzt wurde erst eine Familie mit Mutationen im *Gephyrin-*Gen (GEPH) beschrieben, die zu Moco-Defizienz (Typ C) führten^{B22}.
Als Folge von Mutationen in unterschiedlichen Schritten der Moco-Biosynthese wurde herausgefunden, dass Fibroblasten von Typ B-Patienten Precursor Z anreichern und in das Kulturmedium abgeben; dieser kann von Typ A-Zellen im Zuge einer Co-Kultivierung aufgenommen werden, was zur Wiederherstellung der Mo-Enzymaktivitäten in vivo führt^{B20}. Weiterhin konnte nachgewiesen werden, dass Precursor Z aus menschlichen Fibroblasten mit dem bakteriellen Precursor identisch ist^{B23,B24}, da dessen Biosynthese bei allen Organismen konserviert erfolgt^{B15-B17}.

Aufgabe der Erfindung ist es, ein Mittel zur Therapie humaner Molybdäncofaktor-Defizienz, die direkt oder indirekt auf eine veränderte Molybdäncofaktor-Synthese zurückzuführen sind, bereitzustellen.

Diese Aufgabe wird in Verbindung mit dem Oberbegriff des Anspruches 1 dadurch gelöst, dass als wesentlicher Bestandteil dieses Mittels Precursor Z verwendet wird.

Damit ist es erstmals möglich, ein Mittel zur Therapie humaner Molybdäncofaktor-Defizienz, die direkt oder indirekt auf eine veränderte Molybdäncofaktor-Synthese zurückzuführen sind, bereitzustellen.

Es wird die Gewinnung eines Pterin-Intermediates aus Bakterien, das erfolgreich zur Therapie einer bislang unheilbaren und tödlichen Krankheit eingesetzt wurde, beschrieben.

Der Precursor Z weist C₁₀H₁₄N₅O₈P als Molekülformel auf und stellt ein Pyranopterin dar und trägt ein geminales Diol in der Seitenkette.

Der Precursor Z mit der Molekülformel C₁₀H₁₄N₅O₈P, der ein Pyranopterin darstellt, trägt das geminale Diol in der C1'-Position.

Weitere Einzelheiten der Erfindung ergeben sich aus der nachfolgenden ausführlichen Beschreibung und den beigefügten Zeichnungen, in denen bevorzugte Ausführungsformen der Erfindung veranschaulicht sind.

In den Zeichnungen zeigen:

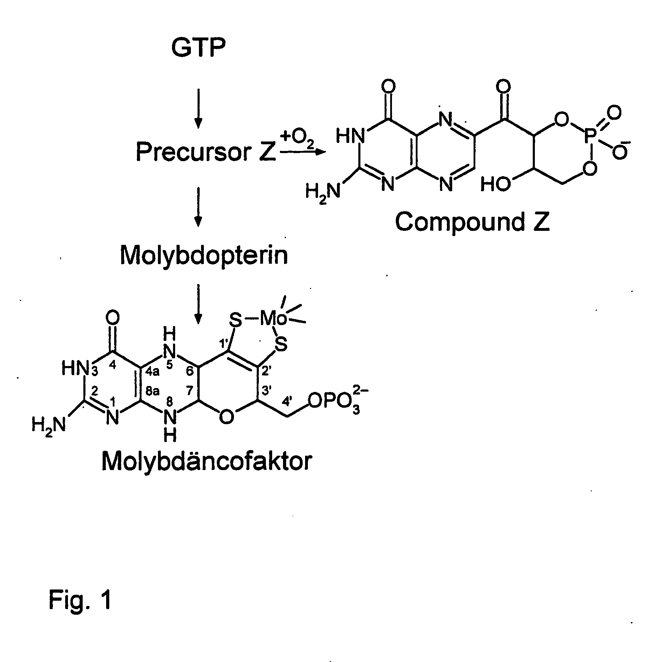

Figur 1: Allgemeines Schema der Moco-Biosynthese. Die Strukturen von Moco (wie in der Kristallstruktur von Mo-Enzymen gefunden) und des Precursor Z-Oxidationsprodukts Compound Z (14,15) sind gezeigt. Nummerierung ist dargestellt und verwendet wie in Standard-Pterin-Nummerierungsschemata, Diskussion siehe (12).

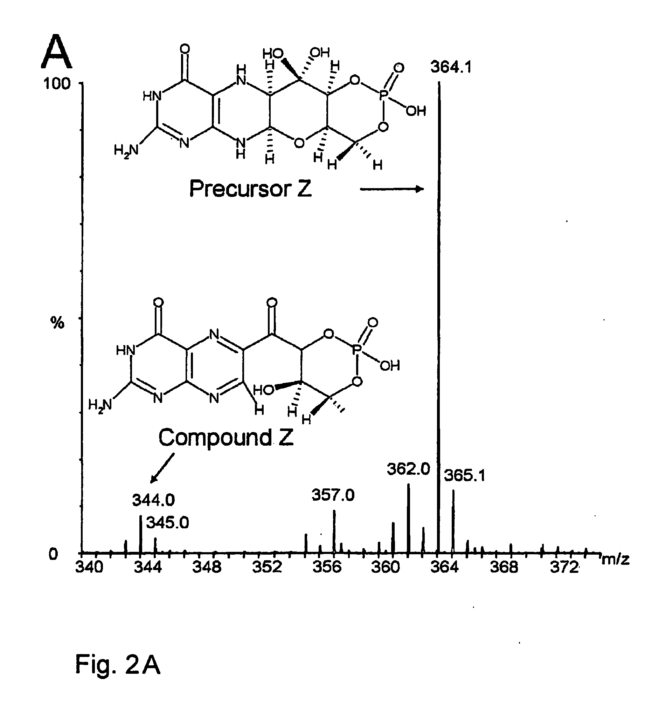

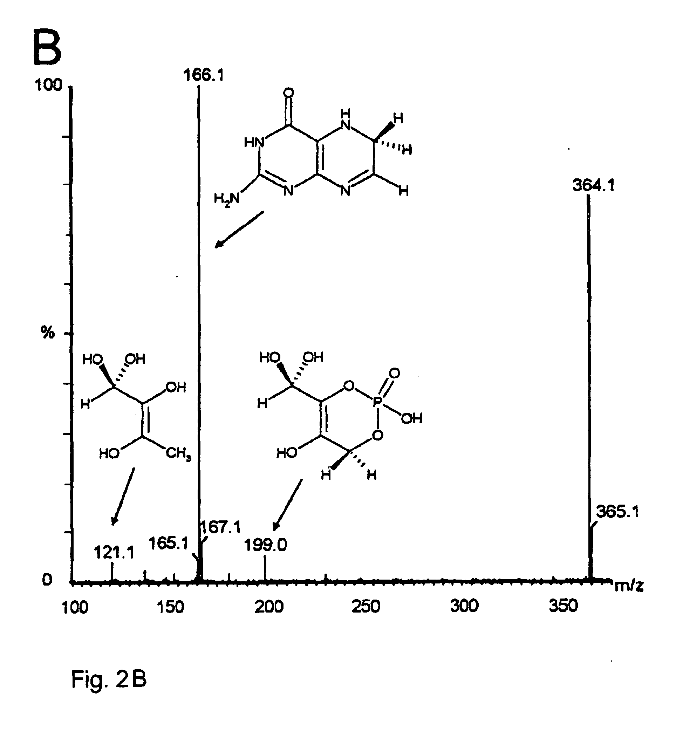

Figur 2: ESI-MS von Precursor Z. A, MS-Spektrum des gereinigten Precursor Z und strukturelle Zuordnung der wesentlichen molekularen Ionen. B, MS/MS-Spektrum des Hauptpeaks bei 364 Da, der in (A) gezeigt wird und strukturelle Zuordnung der Hauptfragment-Ionen. Die Spektren wurden im positiven Ionenmodus aufgenommen. Deshalb sind alle dargestellten Molekül- oder Fragmentionen als neutrale Moleküle und nicht im [M+H]⁺ -Modus wie in den Spektren gezeigt.

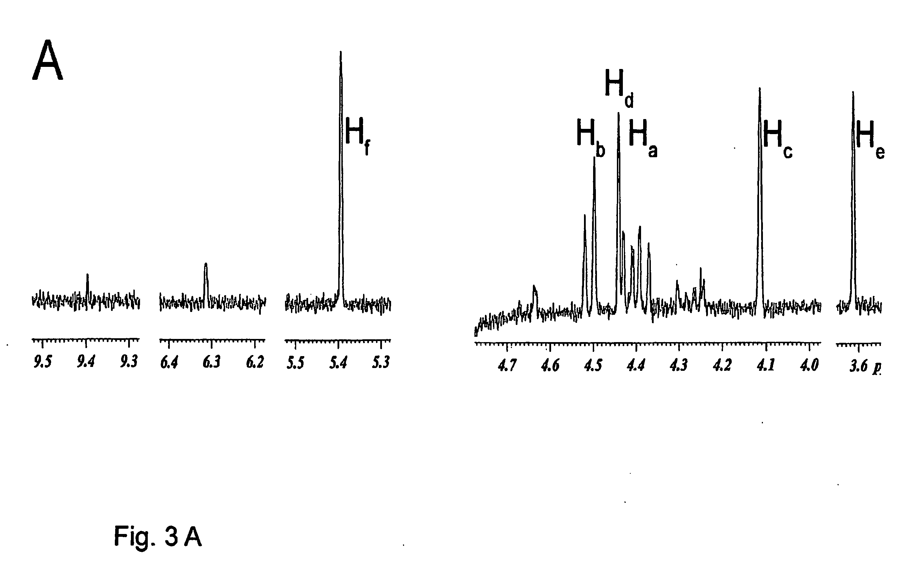

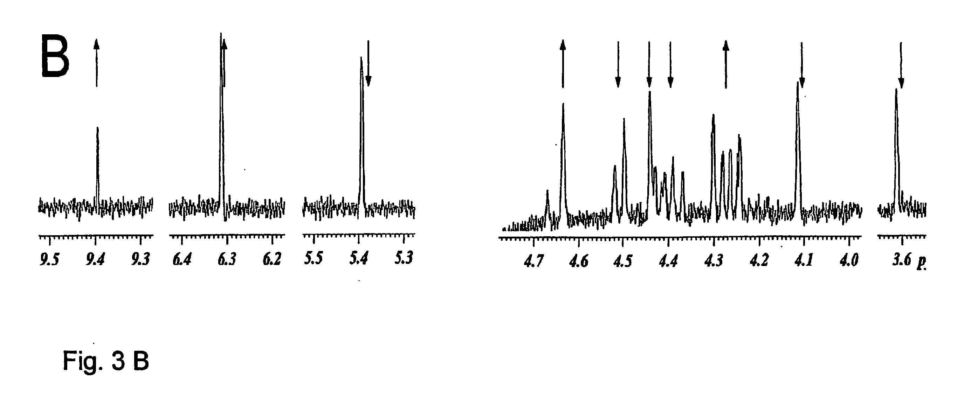

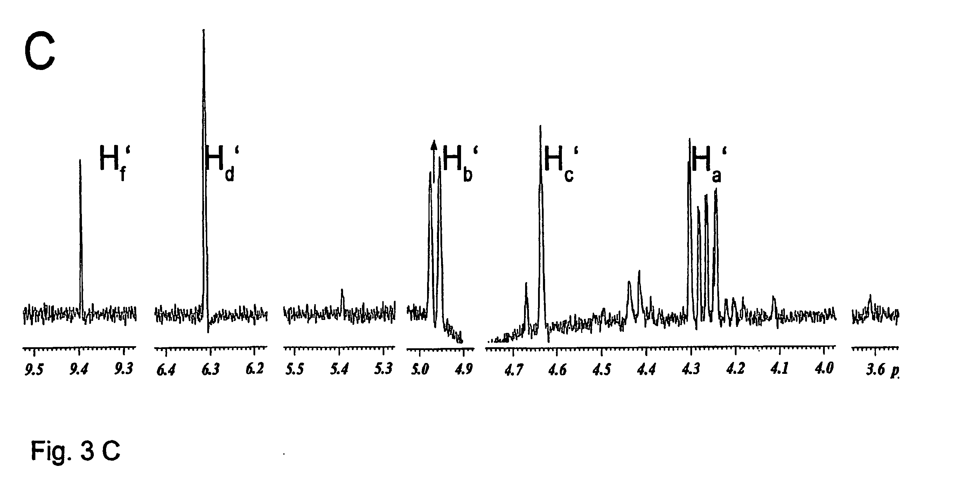

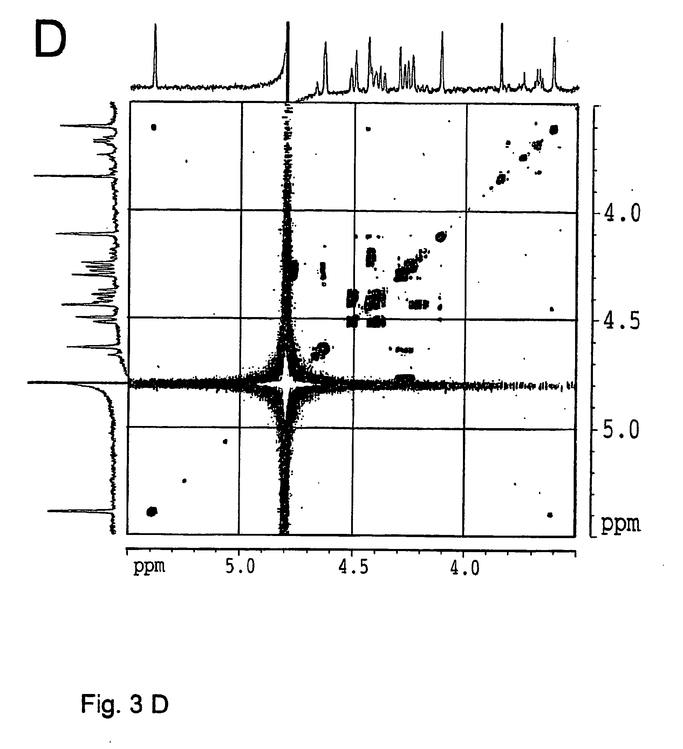

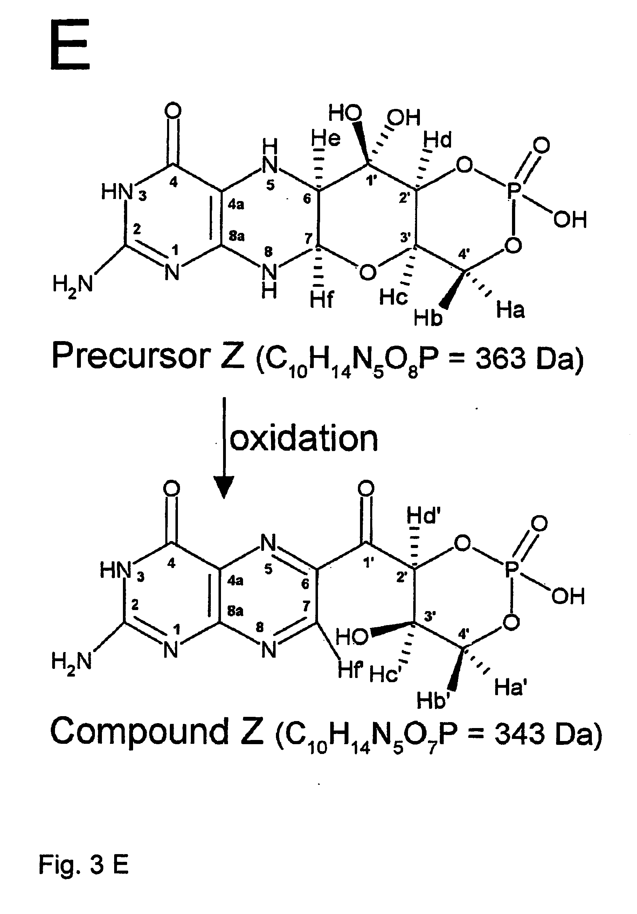

Figur 3: ¹H-NMR- und Korrelations-Spektroskopie bei 300 K von Precursor Z (A), von teilweise oxidiertem Precursor Z (B) und seinem stabilen Oxidationsprodukt Compound Z (C). A-C, Das 1D-¹H-NMR-Spektrum von Precursor Z wurde aufgenommen mit 150 µg Probe, in 700 µl D₂O gelöst und auf pH 1 angesäuert durch Zugabe von 2 µl DCl 36%. Die Messungen wurden nach partieller (14 Stunden, B) und vollständiger Oxidation (14 Tage, C) wiederholt. Die relevanten Bereiche von allen drei Spektren sind dargestellt. Bemerkung: Ein Doublet von Triplet-Signalen von H_{b}' in Coumpound Z liegt unter dem Hintergrundsignal von Wasser bei 300 K, aber es ist bei 315 K deutlich sichtbar (verlgeiche 5,0 - 5,1 ppm in C). D, 2D-COSY-¹H-NMR-Spektrum der teilweise oxidierten Precursor Z-Probe aus (B). E, Struktur des Precursor Z wie sie durch ESI-MS- und ¹H-NMR-Spektroskopie sowie durch die Struktur des Oxidationsprodukts Compound Z bestimmt wurde. Die Benennung der Protonen ist die gleiche wie in A (Precursor Z) und C (Compound Z) sowie in Tabelle 1 und im Text. Compound Z-Protonen sind mit einem Strich markiert, um sie von denen des Precursor Z zu unterscheiden.

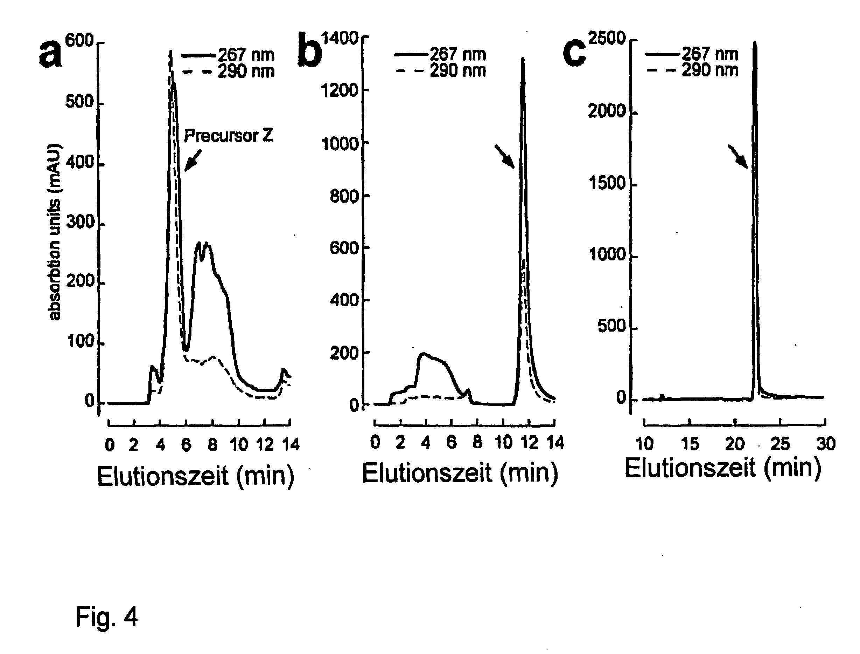

Figur 4: HPLC-Reinigung von Precursor Z aus *E. coli* MJ7 *chlM* (DE3)37 Zellen, die pPH15*moaA* und pPHLys*moaC* enthielten. (a) Elutionsprofil von einer präparativen C8 Säule. (b) Elutionsprofil von einer SAX Säule. (c) Elutionsprofil von einer analytischen C18 Säule. Details siehe Methoden.

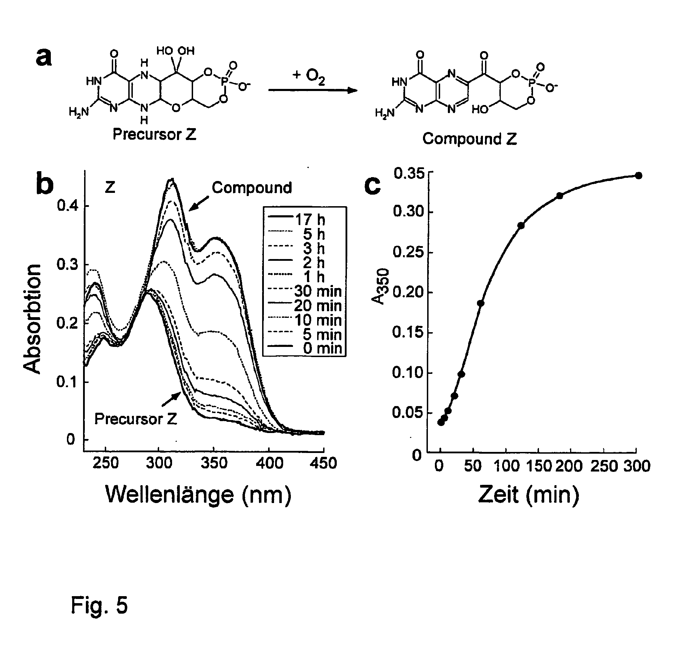

Figur 5: Gereinigter Precursor Z ist unter physiologischen Bedingungen stabil. (a) Oxidation von Precursor Z zu Compound Z. (b) UV-VIS Absorptionsspektren von 22 µM Precursor Z nach Inkubation von 17 h bei Raumtemperatur in 2x PBS-Puffer (pH 6,9). Die Spektren wurden in Zeitintervallen nach Verdünnung in Puffer aufgenommen wie angegeben. (c). Kinetische Analyse der Oxidation von Precursor Z durch Aufnahme der Absorption bei 350 nm aus den in (b) gezeigten Spektren.

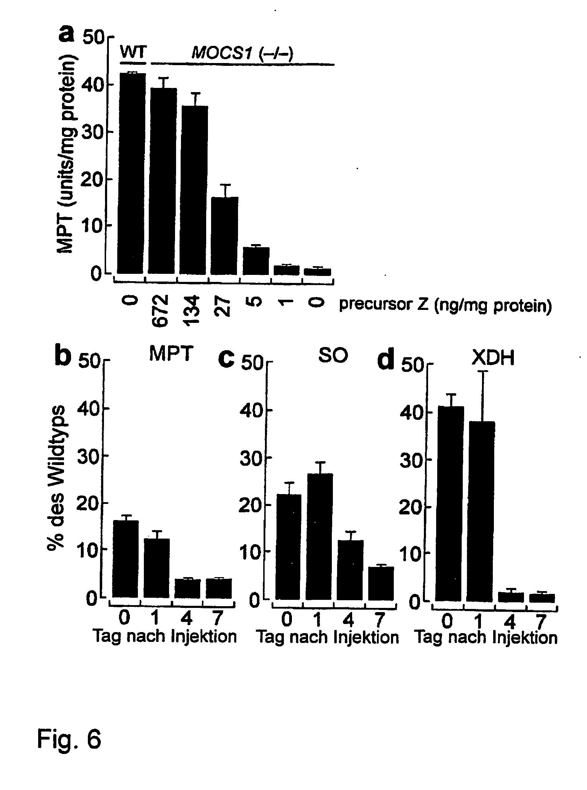

Figur 6: Wiederherstellung der MPT-Biosynthese *in vitro* und in *vivo.* (a) *In vitro* Wiederherstellung von MOCS1-defizienten Leberextrakten mit Precursor Z. Protein-Rohextrakt einer fünf Tage alten *MOCS1* ^{-/*-*} Maus wurde inkubiert (1 h) mit den angegebenen Mengen von gereinigtem Precursor Z pro mg Protein. Wiederherstellung der MPT-Synthese wurde mittels *nit-1* Rekonstitutionsassay gemessen. (b-d) Biochemische Analyse von Precursor Z-behandelten *MOCS1*-defizienten Tieren. Den Mäusen wurden zweimal wöchentlich zunehmende Mengen von Precursor Z injiziert, beginnend mit 2 µg nach der Geburt bis zu 8 µg nach der Entwöhnung. Zwischen den Tagen 25-55 nach der Geburt wurde die Behandlung gestoppt. An den Tagen 0 (4 Stunden nach der letzten Injektion), 1, 4 und 7 wurden jeweils zwei Mäuse getötet. Zwei Leberproben jedes Tieres wurden entnommen und getrennt weiterbearbeitet. Jeder Wert in der Figur wurde somit vierfach bestimmt. Gesamt-MPT einschließlich Moco (b), Sulfit-Oxidase Aktivität (c) und Xanthin-Dehydrogenase (d) sind angegeben als Prozentsatz der entsprechenden Wildtyp-Werte (n=4).

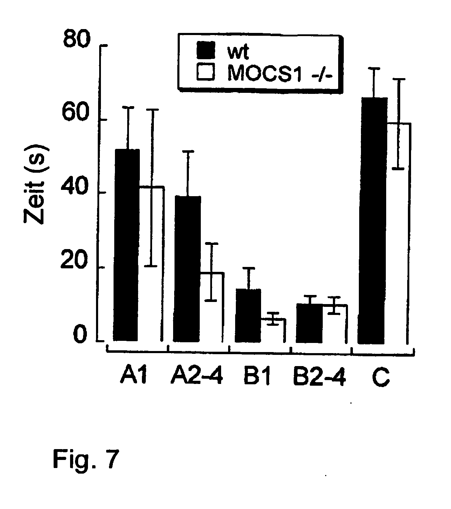

Figur 7: Lernfähigkeit von Precursor Z-behandelten *MOCS1-*defizienten Mäusen in einem Wasserlabyrinth. Die Werte stellen einen Durchschnitt von sechs Wildtyp- (wt, schwarze Balken) und sechs Precursor Z-behandelten *MOCS1*-defizienten Mäusen (MOCS1 ^{-/-}, weisse Balken) dar. Die Balken zeigen die Zeit bis zum Erreichen der Plattform im ersten Versuch des ersten Durchgangs (A1), die verbleibenden drei Versuche des ersten Durchgangs (A2-4), den ersten Versuch des zweiten Durchgangs (B1) und schließlich die folgenden drei Versuche des zweiten Durchgangs (B2-4). C, Zeit (von insgesamt 120 Sekunden), die im richtigen Sektor (ohne Plattform) verbracht wurde, der in den Durchgängen A und B die.

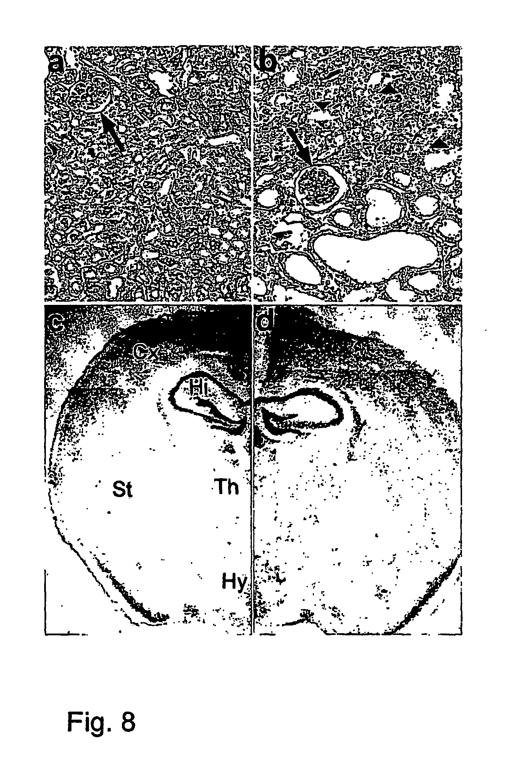

Figur 8: Morphologischer Vergleich von Nieren- (a,b) und Gehirnabschnitten (c,d) von Wildtyp- (a,c) und Precursor Z-behandelten *MOCS1*-defizienten Mäusen (b,d). Behandelt wurde mit 2-4 pg jeden dritten Tag. (a) Reguläre Niere mit normalem Glomerulus (Pfeil), charakteristisch ausgebildeten Tubuli und dünnem Interstitium (200x). (b) Bei behandelten *MOCS1-*defizienten Mäusen enthielten die Nieren zahlreiche Steine in den Tubuli (□) bei atrophen Epithelien und Ausbildung von vielkernigen Riesenzellen (□). Proximale Tubuli waren erweitert (28 +/- 19 %). Das Interstitium wies eine Vergrößerung der Matrix (13 +/- 13 %) und eine unspezifische Infiltrierung mit einkernigen Zellen auf. Der Glomerulus ist normal (Pfeil). (c,d) Entsprechende coronale Gehirnabschnitte (Cx, Cortex; Hi, Hippocampus; Str, Striatum; Th, Thalamus; Hy, Hypothalamus) von wildtyp- (c) und Precursor Z-behandelten *MOCS1*^{-/-} Mäusen (d).

### Spektroskopische Charakterisierung und Strukturaufklärung des gereinigten Precursor Z

Hochauflösende Elektrospray-Ionisations-Massenspektrometrie (ESI-MS) erforderte ein [M+H]⁺ -Ion, welches zur Molekülformel C₁₀H₁₅N₅O₈P kompatibel war. Danach erlaubte die ¹H-NMR-Spektroskopie nicht nur die strukturelle Charakterisierung des Moleküls, sondern bestätigte auch, dass dieses Intermediat direkter Oxidierung zum bereits früher gut charakterisierten, nicht-produktiven Folgeprodukt Compound Z unterliegt. Die chemische ¹H-Verschiebung und die Daten der Kopplungskonstanten stimmen nicht mit früheren Strukturmodellen überein und deuten an, dass Precursor Z bereits als Pyranopterin-System besteht sowie eine zweifache Diol-Funktion in der C1'-Position besitzt.

Der Molybdäncofaktor (Moco) ist Teil des aktiven Zentrums aller Mo-abhängigen Enzyme (1) außer Nitrogenase, und spielt eine wichtige Rolle in den globalen Kohlenstoff- Schwefel- und Stickstoffkreisläufen (2). Mo-Enzyme sind wichtig für diverse metabolische Prozesse wie z.B. Schwefelentgiftung und Purinkatabolismus in Säugetieren (3) sowie Stickstoffassimilierung und Phytohormonsynthese in Pflanzen (4). Der Moco besteht aus einem organischen Teil, der ursprünglich Molybdopterin genannt wurde (5), einem Pyranopterin mit einer terminalen Phosphatgruppe sowie einem Mo-Atom, das über ein En-Dithiol-System gebunden ist (6) (Fig. 1). In allen bisher untersuchten Organismen wird der Moco über einen alten und hochkonservierten Syntheseweg produziert (7). Bei Menschen resultiert eine Mutation in irgendeinem Schritt der Moco-Biosynthese im pleiotropen Ausfall der Aktivitäten von Sulfitoxidase, Aldehydoxidase und Xanthinoxidoreduktase (8,9). Betroffene Patienten weisen neurologische Anormalitäten auf und sterben in früher Kindheit, da bislang keine Therapie vorhanden ist (10). Zusätzlich enthalten viele Prokaryoten, hauptsächlich Archaea, Metallenzyme ähnlichen Pyranopterin-En-Dithiolat-Cofaktor, der W statt Mo koordiniert. Deshalb wird die grundlegende Pyranopterinkomponente von Mo- und W-Enzymen als MPT abgekürzt, welches für MolybdoPTerin oder Metall-bindendes Pyranopterin-DiThiolen steht (11,12). Es wird ebenso vermutet, dass die W-Cofaktor-Biosynthese, der Moco-Biosynthese ähnlich ist, den finalen Schritt der Metallinsertion ausgenommen (7).
Die Moco-Biosynthese (Fig. 1) kann in drei Schritte unterteilt werden (7,13). Zuerst wird der Schwefel-freie Precursor Z aus einem Guanosinderivat durch die Aktion von zwei Proteinen (14,15) synthetisiert. Im zweiten Schritt wird Precursor Z durch den Einbau von zwei Schwefelatomen an den C1'- und C2'-Positionen zu MPT umgewandelt (siehe neue Nummerierung in Fig. 1), wodurch eine En-Dithiol-Funktion gebildet wird (16). Diese Reaktion wird von der heterotetrameren MPT-Synthase katalysiert (17), die zwei Schwefelatome von zwei thiocarboxylierten kleinen Untereinheiten auf die Kohlenstoffatome C1' und C2' überträgt (18,19). Im letzten Schritt der Moco-Biosynthese wird ein Mo-Atom auf ein (Pro- und Eükaryoten) oder zwei (Prokaryoten) MPT-Dithiolengruppen übertragen, was zur Bildung des Moco führt (20, 21). In Bakterien wurde eine zusätzliche Modifizierung des Pterinphosphats durch Anhängen eines Nukleotids beobachtet (22). Die grundlegende chemische Struktur des Moco wurde durch die Pionierarbeit von Rajagopalan und Mitarbeitern (13,23) aufgeklärt. Ihre letzte Beschreibung des Moco wurde durch die Kristallstrukturen von Mo- (24) und W-haltigen Enzymen aufgeklärt (25), bei denen ein nicht vorhergesehener zusätzlicher Pyranoring entdeckt wurde, der zwischen der Hydroxylgruppe des C3'-Atoms und der C7-Position des Pterins gebildet wird (Fig. 1). Basierend auf dieser Beobachtung wurde vermutet, dass der Pyranoring entweder bereits in den Intermediaten vorhanden ist (12,18,26), oder dass er während der Insertion in das Apoenzym gebildet wird (27). Bisher wurde weder MPT noch Precursor Z mittels ¹H- oder ¹³C-NMR-Spektroskopie untersucht oder kristallisiert. Deshalb bleibt die Frage offen, ob die Pterin-Seitenkette bereits in einem frühen Schritt der Moco-Biosynthese eine Pyranopterinstruktur bildet oder nicht.

Das Strukturmodell von Precursor Z wurde basierend auf der Struktur des Oxidationsprodukts (28), auf ³¹P-NMR-Spektroskopie, auf Massenspektrometrie (MS) sowie Oxidationsstudien aufgestellt (14). Die direkte stöchiometrische Konversion von Precursor Z durch eine Zwei-Elektronen-Oxidation zum stabilen Oxidationsprodukt Compound z implizierte einen Dihydro-Zustand von Precursor Z, der durch eine chinonoide Struktur und eine Enol-Funktion in C1' erklärt wurde, welche einer Keto-Enol-Tautomerisierung unterliegen könnte (14). Precursor Z ist ebenso wie Compound Z schwefelfrei und enthält ein zyklisches Phosphat, das C2' und C4' verbindet. Im Vergleich zu MPT und Moco ist Precursor Z das stabilste Intermediat mit einer geschätzten Halbwertzeit von einigen Stunden bei niedrigem pH-Wert (14).

Deshalb haben wir eine Methode für die Reinigung großer Mengen Precursor Z aus *E.coli* etabliert. Wir berichten von einer detaillierten spektroskopischen Analyse und strukturellen Charakterisierung des Precursor Z. Durch ¹H-NMR- und MS-Methoden zeigen wir, dass Precursor Z bereits eine vollständig reduzierte Pyranopterinstruktur besitzt und vorrangig an der C1'-Position hydriert ist, was zu einem zweifachen Diol führt.

### Chemikalien

NaCl, Hefe, Trypton und Isopropylthiogalactosid stammen von Duchefa Biochemie, Ammoniumacetat von Merck, Zitronensäure-Monohydrat und Natriumcitrat-Dihydrat von Baker, Ameisensäure von Sigma, D₂O und DCl waren,von Deutero GmbH und NaOD von der Fluka AG.

### Konstruktion der Plasmide für die Coexpression von MoaA und MoaC

*E. coli moaA* und *moaC* wurden mittels PCR aus pJR11 kloniert (29). Die veröffentlichte Gensequenz (30) wurde für die Herstellung von Oligonukleotiden genutzt, die die Klonierung in die *Nde* I- und *Xho* I-Schnittstellen der Klonierungsregion des pET15b-Expressionsvektors (Novagen) ermöglichten. Die entstandenen Plasmide wurden pPH15*moaA* und pPH15*moaC* genannt. Die vollständige *moaC*-Expressionseinheit inklusive des IPTGregulierten T7-Promotor/Operator-Elements und der synthetischen Ribosomen-Bindungsstelle von pPH15*moaC* wurde anschließend in die *Sph* I- und *Hind* III-Schnittstellen von pLysS (Novagen) subkloniert, woraus der pPHLys*moaC* entstand.

### Isolierung und Reinigung von Precursor Z

Precursor Z wurde aus *E. coli* mittels eines modifizierten Protokolls gereinigt, das bereits beschrieben wurde (14). MJ7 *clM* (DE3)-Zellen (31), die die Plasmide pPH15moaA und pPHLysmoaC enthielten, wurde anaerob bei 20°C in LB-Medium mit 120 µg/ml Ampizillin, 30 µg/ml Chloramphenicol und 50 µM IPTG angezogen und mittels Zentrifugation (5 min, 12000 g, 4°C) geerntet. Vor der HPLC-Reinigung wurden die Zellen in zwei Volumina 0,4 M HCl resuspendiert, sonifiziert und zentrifugiert. Um etwaige Modifikationen nachzuweisen, die durch die Azidifizierung der Zellen entstehen könnten, wurden die Zellen in einem Kontrollexperiment in 100 mM Acetat-Puffer (pH 3,0) extrahiert. Der klare Überstand wurde auf eine semi-präparative "Reversed-Phase"-Säule (C8, 5 µm, 250*10 mm, Kromasil, EKA Chemicals) gegeben, die in 5 mM Ammoniunacetat, pH 5,0, äquilibriert worden war. Precursor Z eluierte im ersten Absorptionmaximum und wurde sofort in flüssigem Stickstoff eingefroren. Im zweiten Schritt wurden die Precursor z-haltigen Fraktionen vereinigt und auf eine semi-präparative SAX-Säule (15 µm, 250 x 10 mm, Adsorbosphere, Alltech) gegeben, die in 10 mM Citratpuffer, pH 3,0, äquilibriert worden war. Precursor Z wurde nach etwa 30 ml isokratisch eluiert und in flüssigem Stickstoff eingefroren. Eine abschließende Reinigung wurde durch das Auftragen von Precursor Z auf eine analytische "Reversed-Phase"-Säule (C18, 5 µm, 250 x 4 mm, Alltech), äquilibriert in 10 mM Ameisensäure, erzielt. Precursor Z-haltige Fraktionen wurden vereinigt, in 20 µl Aliquots in flüssigem Stickstoff eingefroren und bis zum weiteren Gebrauch bei -80°C gelagert. Konzentrationen von Precursor Z wurde über den Extinktionskoeffizienten □₂₆₇ₙₘ= 8960 M cm⁻¹ (14) bestimmt.

### Elektrospray-Massenspektrometrie

Precursor Z wurde in einem 1:1 (Vol:Vol) Methanol / 1% Ameisensäure -Gemisch gelöst. Etwa 3 µl dieser Lösung (Endkonzentration ca. 20 pmol/µl) wurden in eine Gold-beschichtete Nanospray-Kapillare (Protana) gegeben. Die Spitze der Kapillare wurde senkrecht vor die Eintrittsöffnung eines "quadrupole-time-of-flight" (QTOF 2)-Massenspektrometer (Micromass) platziert, das mit einer Nanospray-Ionenquelle ausgestattet war, und es wurde eine Spannung von etwa 1000 V angelegt. Für Kollisions-induzierte Dissoziationsexperimente wurden Parental-Ionen selektiv vom Quadrupol-Massenanalysator in die Kollisionszelle transmittiert. Als Kollisionsgas wurde Argon genutzt, und die kinetische Energie wurde auf etwa -25 eV festgelegt. Die resultierenden Schwester Ionen wurden anschließend durch den orthogonalen "time-of-flight"-Massenanalysator aufgetrennt. Die isotopische Zusammensetzung der Probe wurde im akkuraten Massenmodus bestimmt, der Reserpin ([M+H]⁺ = 609.2811 Da) als internes Referenzmolekül verwendet.

### ¹H-NMR-Spektroskopie

Ein- (1D) und zweidimensionale (2D) ¹H-NMR-Korrelationsspektren (COSY) wurden bei 300 K mit einem Bruker Avance DMX600 NMR-Spektrometer aufgenommen, der an die überwiegende Resonanz von Deuterium aus dem Lösungsmittel D₂O gebunden ist. Chemische Verschiebungen sind in ppm relativ zum Hintergrundsignal des Lösungsmittels (4.80 ppm) angegeben und Kopplungen in Hz. Precursor Z-haltige Proben (100-200 µg) wurden gefriergetrocknet und in 700 µl entgastem D₂O und 2 µl DCl 36% resuspendiert. Zur Vermeidung von Oxidation wurde das Röhrchen für 5 min mit Stickstoffgas begast. Diese Lösungen waren stabil genug für die Aufnahme von sowohl 1D- als auch 2D-COSY-Spektren. Anschließendes Exponieren der Lösung an Luft verursachte die Oxidation des Precursor Z zum gut beschriebenen Haupt-Einzelprodukt, Compound Z, und diese Reaktion wurde in regelmäßigen Intervallen durch die Aufnahme von 1D- ¹H-Spektren verfolgt. Unter normalen Bedingungen ist diese Reaktion nach 14 Tagen in D₂O und DC1 abgeschlossen.

### Ergebnisse

### Isolierung und Reinigung von Precursor Z

Precursor Z wurde aus *E. coli* isoliert und in einer dreischrittigen HPLC-Chromatographie-Prozedur gereinigt, mit der ein homogenes Produkt erzielt wurde, welches durch UV-Vis (Daten nicht gezeigt), ESI-MS und ¹H-NMR-Spektroskopie (siehe unten) nachgewiesen wurde. Durchschnittlich wurde ein Ertrag von 40 µg Precursor Z pro Liter *E. coli*-Kultur erzielt. Das isolierte Molekül war identisch mit früheren Präparationen (12), da es das gleiche UV-Vis-Absorptionsspektrum mit einem Absorptionsmaximum bei 267 nm bei pH 3,0 aufwies und nach Aussetzung an Luft das gleiche, eindeutig eindeutig charakterisierte Oxidationsprodukt, Compound Z, zur Folge hatte.

### ESI-MS von Precursor Z

ESI-MS wurde angewendet, um die Molekülmasse des Precursor Z zu bestimmen. Im positive Ionenmodus ergab die ESI-MS ein [M+H]⁺ Ion bei 364 Da (Fig. 2A), welches 18 Da größer war als die erwartete Masse von 346 Da der früheren Modellstruktur (14). Tatsächlich gab es keinen Hinweis auf einen solchen Peak im Spektrum. Ein weiterer kleiner Peak im Spektrum (Fig. 2A) bei 344 Da entsprach der erwarteten Masse für Compound Z (14), welcher in solchen Proben aufgrund partieller Oxidation unausweichlich vorhanden ist (Fig. 2A). Diese Tatsache wurde auch durch ¹H-NMR-Analyse bestätigt (siehe unten). Im Gegensatz zu früheren Versuchen mit Thermospray oder "fast atom bombardment ionization" konnten wir im positiven Ionenmodus mittels ESI-MS mit 364.064±0.003 Da (364.0658 Da berechnet für C₁₀H₁₅N₅O₈P) eine genaue Masse für Precursor Z bestimmen. Dementsprechend ist die Molekülformel von Precursor Z C₁₀H₁₄N₅O₈P, was nicht mit der früher postulierten Formel C₁₀H₁₂N₅O₇P übereinstimmt (12). Die neue Formel des 364 Da-Peaks lässt eine Hydratisierung des Precursor Z vermuten, die am wahrscheinlichsten an der C1'-Position stattfindet, für die bereits eine einzelne Hydroxylierungsfunktion mit einer möglichen Keto-Enol-Tautomerisierung vorgeschlagen wurde (14). Die zusätzliche Hydratisierung am C1' würde dann in der Bildung eines zweifachen Diols resultieren (Fig. 2A).
Eine MS/MS des 364 Da-Peaks zeigte ein Hauptfragment bei 166 Da (Fig. 2B), welches einem semi-oxidierten Dihydropterin entspricht und auf die Pterinnatur des Peaks hinweist. Das Dihydropterin kann nach der Öffnung des Pyranorings ohne weitere Oxidation einfach gebildet werden (vergleiche mit Moco in Fig. 1). Zwei weitere Peaks wurden bei 121 Da und 199 Da beobachtet, die jeweils den Massen der Seitenkette ohne bzw. mit dem zyklischen Phosphat entsprechen. Beide Massen passen nur für hydrierte Seitenketten-Fragmente, die ein zweifaches Diol in der C1'-Position besitzen. Außerdem ergab MS/MS des 344 Da-Peaks in der hinteren Probe oder in der Probe von gereinigtem Compound Z eine ähnliches Schwester Ionen Spektrum mit einem dominierenden Fragment bei 164 Da (Daten nicht gezeigt), welches einem vollständig oxidierten nichtsubstituierten Pterin entspricht, das mit MS/MS-Daten von anderen hydrogenierten Pterinen vergleichbar ist (32).

### ¹H-NMR-Spektroskopie von Precursor Z

Um die Struktur des Precursor Z aufzuklären und um unsere Hypothese der Hydratisierung zu festigen, die bei der MS beobachtet wurde, nahmen wir ¹H-NMR-Spektren in azidifiziertem D₂O auf und verfolgten die Änderung der Signalpositionen und -intensitäten während der Oxidation zu Compound Z (Fig. 3A-D). Auf Basis der ¹H chemischen Verschiebungen (Fig. 3A-C), der Verbindungen der 2D COSY-Spektren (Fig. 3D) und der Größenordnung der Kupplungskonstanten wurden alle erwarteten Signale (sechs Protonen, Hₐ-H_{f}) eindeutig zugeordnet (Tab. 1). Die ¹H chemischen Verschiebungen und Kupplungskonstanten (¹H-¹H und ³¹P-¹H) des Oxidationsprodukts Compound Z waren vergleichbar mit den veröffentlichten Daten (15,28) und identifizierten diesen zweifelsfrei als Compound Z (Fig. 3C).
Die drei ¹H-NMR-Signale der Protonen Hₐ, H_{b} , und H_{c} in Precursor Z sind in Bezug auf die chemischen Verschiebungen sowie die ¹H-¹H und ¹H-³¹P Kupplungskonstanten (Fig. 3 und Tab. 1) vergleichbar mit den Protonen, die im Spektrum von Compound Z (Hₐ', H_{b}', and H_{c}') vorhanden sind. Diese drei Protonen wurden C4' (Hₐ und H_{b}) und C3' (H_{c}) zugeordnet, und die Größenordnung der benachbarten ¹H-³¹P-Kupplungen der C4'-Protonen (22.7 bzw. 2.1 Hz) wiesen auf das intakte zyklische Phosphat hin (12). Die größte Verschiebung von H_{c} unter diesen drei Protonen deutet signifikante Änderungen in der Umgebung von C3' an, die durch die Öffnung der vorgeschlagenen Pyranopterin-Ringstruktur in Precursor Z während der Oxidation zu Compound Z erklärt werden könnten.
Die Signale der übrigen drei Protonen (H_{d}, Hₑ, and H_{f}) von Precursor Z weichen signifikant von denen von Compound Z (H_{d}', Hₑ', und H_{f}', Fig. 3 und Tab. 1) ab. Precursor Z zeigt ein 1.7 Hz-Dublett bei 5.39 ppm (H_{f}), das mit dem breiten Dublett (breites Signal) bei 3.62 ppm (Hₑ) korreliert. Diese beiden Signale gehen allmählich während der Konversion zu Compound Z verloren, während gleichzeitig das Signal bei 9.39 ppm (H_{f}, Fig.3A-C) erscheint. Die chemische Verschiebung der H_{f} - und Hₑ -Signale ist vergleichbar mit den wenigen chemisch synthetisierten Pyranopteridinen (33). Unabhängig von den hochaufgelösten ESI-MS-Daten sprechen die Kupplungskonstanten und eine große Differenz in den chemischen Verschiebungen dieser zwei Protonen gegen das frühere Strukturmodell, da sie zu einer Stickstoff-substituierten Methylgruppe gehört hätten. Das dritte wichtige Signal zeigt ähnliche benachbarte Kupplungen zu H_{c} und P sowohl in Precursor Z als auch Compound Z, aber verschiebt sich während des Oxidationsprozesses feldabwärts von 4.415 ppm zu 6.32 ppm (Fig. 3 und Tab. 1). Diese Daten implizieren die gleiche relative Anordnung von H_{d} im Vergleich zu H_{c} und P in beiden Verbindungen. Die 1.87 ppm Differenz in der chemischen Verschiebung des H_{d} Signals favosisiert sehr einen signifikanten Wechsel im Substitutenten an C1' zwischen Precursor Z und Compound Z, wodurch die Möglichkeit einer unveränderten Ketofunktion in beiden Komponenten ausgeschlossen wird. Zusätzliche ¹H-NMR-Spektren wurden bei verschiedenen pH-Werten (1, 3, 7 und 10) aufgenommen ohne signifikante Änderungen in den Protonensignalen von Precursor Z zu beobachten (Daten nicht gezeigt). Die einzige bemerkenswerte Differenz bei höheren pH-Werten war die Verbreiterung der Hₑ und H_{f} Signale, die mit einer schnelleren Oxidierung zu Compound Z einherging. Somit sind die hochauflösenden ESI-MS- und ¹H-NMR-Daten für Precursor Z zusammen mit den beobachteten Änderungen bei der Oxidation zu Compound nur mit der geschlossenen Pyranopterinstruktur und einem zweifachen Diol in C1' (Fig. 4E) kompatibel.

**Tabelle 1. ¹H-MMR-Signale von Precursor Z und Compound Z bei pH 1 in D₂O / DCl.**

| **¹H-Signale** | **Chemische Verschiebungen (ppm)*** | **Kopplungskonstanten (Hz) J (¹H-¹H, ³¹P- ¹H)** | **COSY** |
|---|---|---|---|
| Precursor Z | | | |
| Hₐ | 4.41 (ddd) | 22.7 (³¹P), 13 (H_{b}), 1. 9 (H_{c}) | H_{b}, H_{c} |
| H_{b} | 4.51 (dt) | 13 (Hₐ), 2.1 (³¹P), 2.0 (H_{c}) | Hₐ, H_{c} |
| H_{c} | 4.12 (q) (br) | 1.9 (Hₐ), 1.9 (H_{b}), 1.9 (³¹P), 1. 9 (H_{d}) | Hₐ, H_{b}, H_{d} |
| H_{d} | 4.45 (t) (br) | -1.6 (³¹ P), 1.6 (H_{c}), -0.8 (Hₑ) | H_{c}, Hₑ |
| Hₑ | 3.62 (d) (br) | ~1.7 (H_{f}), -0.8 (H_{d}) | H_{f}, H_{d} |
| H_{f} | 5.39 (d) | 1.7 (Hₑ) | Hₑ |

| Compound Z | | | |
|---|---|---|---|
| Hₐ' | 4.26 (ddd) | 22.7 (³¹P), 12.5 (H_{b}), 1.9 (H_{c}) | H_{b}, H_{c} |
| H_{b}' | 4.80 (dt) 4.97 (dt) at 315 K | 12.5 (Hₐ), 1.9 (³¹P), 1.9 (H_{c}) | Hₐ, H_{c} |
| H_{c}' | 4.64 (q) | 1.9 (Hₐ), 1.9 (H_{b}), 1.9 (³¹P), 1.9 (H_{d}) | Hₐ, H_{b}, Hd |
| H_{d}' | 6.32 (t) | 1.6 (³¹P), 1.6 (H_{c}) | H_{c} |
| Hₑ' | - | - | - |
| H_{f}' | 9.39 (s) | - | - |

| | | | |
|---|---|---|---|
| *Chemische Verschiebungen bei 300 K aufgenommen, wenn nicht anders angegeben. Kopplungskonstanten sind in Klammern angegeben: s = Singlet, d = Dublett, t = Triplett, q = Quintett. Breite Peaks sind mit (br) gekennzeichnet. | | | |

### Diskussion

Precursor Z wurde zuerst von Wuebbens und Rajagopalan (14) identifiziert und als Dihydropterin-Struktur mit einer C6-substitutierten Vier-Kohlenstoff-Seitenkette sowie einer Hydroxylfunktion in C1' beschrieben. Basierend auf dem Dihydro-Zustand und der relativen Stabilität des Precursor Z wurde eine chinonoide Struktur des Precursor Z mit einer möglichen Keto-Enol-Tautomerisierung in der C1'-Positon postuliert (14). Neue Einblicke in die Struktur des Moco wurden aus den Kristallstukturen der Mo-Enzyme abgeleitet (24,34,35) und warfen die Frage auf, ob ein nicht-koordiniertes MPT und/oder Precursor Z bereits eine Pyranopterin-Struktur besitzen (12). In der hier präsentierten Studie haben wir uns dieser Fragestellung zugewandt und die Seiterikettenstruktur des Precursor Z durch die Anwendung von MS und ¹H-NMR-Spektroskopie auf ausreichend große Mengen gereinigten Precursors Z beschrieben und verifiziert.
Ein Ziel dieser Studie war es, den Öxidationszustand des Precursor Z Pterin-Systems zu etablieren, um seine Rolle im Zusammenhang mit dem Biosyntheseweg aufzuklären. Die Beobachtung von zwei Protonen an C6 (Hₑ) und C7 (H_{f}), welche eine benachbarte Kopplung von 1.7 H_{z} (COSY) und eine große chemische Verschiebungsdifferenz zeigen, impliziert, dass Precursor Z im Pyrazinring des Pterins vollständig hydriert ist. Ein Dihydropterin würde eine Doppelbindung entweder zwischen N5 und C6 (7,8-dihydro), C6 und C7 (5,8-dihydro) oder N8 und C7 (5,6-dihydro) erfordern. Im Falle eines 7,8- oder 5,8-Dihydropterins würde das Hₑ -Proton verloren gehen, während für das 5,6-Dihydropterin die Anwesenheit einer Doppelbindung zwischen C7 und N8 wegen des Rückzugseffekts der Doppelbindung, wie es schon in anderen Dihydropterinen beobachtet wurde (36)., in einer substanziellen Verschiebung zu einer Niedrigfeld-Resonanz des H_{f} -Signals (~7.5 ppm) führen würde. Desweiteren ist die chemische Verschiebungsdifferenz zwischen beiden Pterinprotonen (Hₑ and H_{f}) von 1.77 ppm sehr ähnlich zu denen, die in den wenigen Berichten über chemisch synthetisierte Pyranopteridine (Hₑ =3.1 ppm, H_{f} =4.8 ppm in DMSO) beschrieben wurden (33).

Ebenso war es wichtig festzustellen, ob es eine Doppelbindung zwischen C1' und C2' gibt, wie sie in MPT und Moco gefunden wurde. Die Präsenz des Precursor Z Signals bei 4.41 ppm (H_{d}), welcher die gleiche Kopplungspartner wie in Compound Z aufzeigt, erlaubt die Zuordnung des Protons an der C2'-Position. Demnach schließt die Präsenz von Protonen an C2' und C6 die Anwesenheit von Doppelbindungen entweder zwischen C6 und C1' (14) oder C1' und C2' (26) aus. Da die ¹H-NMR-Daten nicht auf die Anwesenheit eines Protons an C1' hindeuteten, war es möglich, dass eine Carbonylgruppe an C1' positioniert ist; vergleichbar mit derjenigen in Compound Z (15, 28). Sowohl das hochauflösende ESI-MS-Massenspektrum des Precursor Z als auch die große Differenz in der chemischen Verschiebung (1.87 ppm) für das C2'-gebundene H_{d} -Proton zwischen Precursor Z und Compound Z konnten nicht mit der Aromatisierung des Pterinrings bedingt durch die Oxidation zu Compound Z erklärt werden, sondern erfordern zusätzlich eine Änderung in den Substituenten an C1' durch die Bildung eines zweifachen Diols. Weil alle ¹H-NMR und ESI-MS-Spektren bei sauren pH-Werten von 1-3 aufgenommen wurden, könnte das zweifache Diol das Produkt einer *in vitro* Hydratisierung sein, die unter sauren Bedingungen katalysiert wird. Um festzustellen, ob eine Carbonylgruppe unter verschiedenen pH-Bedingungen in einer Gleichgewichtsreaktion gebildet wird, wurden ¹H-NMR-Spektren bei verschiedenen pH-Werten (1-10) aufgenommen. Der einzige in den Spektren beobachtbare Unterschied war die Verbreiterung der Signale von Hₑ Und H_{f}, während H_{d} keine Veränderungen zeigte. Daraus können wir schließen, dass das zweifache Diol bei allen pH-Werten das Hauptprodukt ist. Hydration an der C1'-Position der Seitenkette scheint eine gemeinsames Merkmal bei Tetrahydropterinen zu sein, da bei einem Intermediat (6-Pyruvoyl-5,6,7,8-Tetrahydropterin) in der Biosynthese von Tetrahydropterin ein zweifaches Diol an der C1'-Position mit mehr als 90 % Hydroxylierung bei neutralem pH nachgewiesen wurde (37). Neben einer *in vitro-* Hydratisierung könnte die Möglichkeit einer *in vivo-* Hydratisierung während der Synthese von Precursor Z bestehen. Daraus schließen wir, dass das zweifache Diol in Precursor Z und anderen reduzierten Pterinen als eine Schutzfunktion dienen könnte. Bisher haben die für den Mechansimus der Precursor Z-Biosynthese aufgestellten Theorien (15,38,39) weder die Bildung eines zweifachen Diols noch die Syntheses eines Pyranopterins in Betracht gezogen. Es wurde postuliert, dass die Precursor Z-Bildung durch eine alternative Cyclohydroxylase-ähnliche Reaktion mit einem Guanosin-Derivat als Startkomponente abläuft (14,15). Während der Precursor Z-Biosynthese werden alle Kohlenstoffatome des Guanosins benutzt, weil das Imidazolring-C8-Atom erhalten bleibt und in einer Umordnungsreaktion als C1' in Precursor Z inkorporiert wird (14,15). Für diese Reaktion wird die Bildung eines transienten Formylesters diskutiert (15), welcher an den konservierten und functional wichtigen C-Terminus von Proteinen der MOCS1A-Familie gebinden sein könnte (40). Das zweifache Diol könnte das Produkt der Formyl-Freisetzungsreaktion während der Precursor Z-Synthese sein. Neben der erhöhten Stabilität könnte das zweifache Diol eine zweite biosynthetische Funktion besitzten. Wir haben gezeigt, dass *E. coli* MPT-Synthase zwei Schwefelatome von zwei verschiedenen thiocarboxylierten kleinen Untereinheiten auf Precursor Z überträgt, was auf die Bildung eines Ein-Schwefel-Intermediats hinweist (18), dessen Existenz vor Kurzem gezeigt wurde (26). Die Übertragung des ersten Schwefels geht mit der Öffnung des zyklischen Phosphats einher, was den initialen Angriff an der C2'-Position andeutet. Letzteres ergibt Sinn in Hinsicht auf die niedrigere Reaktivität des zweifachen Diols an C1' verglichen mit einer Keto-Funktionalität. Der zweite Schritt der Reaktion würde die Freisetzung beider Hydroxylgruppen sowie die Übertragung des zweiten Schwefelatoms involvieren.
Zusammenfassend schließen wir, dass die grundsätzliche Struktur des Precursor Z ein Pyranopterin ist. Diese Entdeckung zeigt, dass nicht nur der Moco sondern auch beide Intermediate des Biosynthesewegs (Precursor Z und MPT) als Pyranopterine vorliegen und dass die Öffnung dieses Pyranoringmerkmals mit hoher Wahrscheinlichkeit alle Zwischenprodukte inclusive des Moco selbst irreversibel zerstören wird. Sowohl die Pyranoring-Bildung als auch die beobachtete zweifache Diol-Funktionalität in C1' scheint wichtig zu sein, um (i) Precursor Z vor Oxidation zu schützen, (ii) die Stereochemie der Pterin-C6-Position zu erhalten und (iii) für die gerichtete Reaktivität des Precursor Z bei der MPT-Synthese zu sorgen.

### Heilung von tödlicher Molybdän-Cofaktor-Defizienz durch einen biosynthetischen Precursor aus Escherichia coli

Wir haben jüngst die Konstruktion eines tierischen Modells für humane Moco-Defizienz Typ A beschrieben^{B25}. Diese *MOCS1* Knock-out Mäuse zeigen einen schweren Phänotyp, der mit den biochemischen Charakteristika humaner Moco-Defizienz Patienten übereinstimmt. Die Mäuse sind im Wachstum gehemmt und sterben innerhalb der ersten 12 Tage nach der Geburt, bei einer durchschnittlichen Lebensspanne von 7,5 Tagen.

Dieser lethale Phänotyp kann erfindungsgemäß durch ein biosynthetisches Intermediat, Precursor Z aus *E*. *coli,* wirkungsvoll behoben werden kann.

### Ergebnisse

### Reinigung und Stabilität von Precursor Z

Precursor Z wurde durch einen drei Schritte umfassenden HPLC Chromatographie-Verfahren bis zur Homogenität gereinigt (Fig. 4), was sowohl mittels UV-VIS Spektroskopie (Fig. 5b) als auch anhand von Massenspektrometrie demonstriert werden konnte (Daten nicht gezeigt). Um größere Mengen zu erhalten, benutzten wir einen Bakterienstamm, der Precursor Z aufgrund eines Defektes in der Umwandlung zu MPT anreichert. Eine weitere Verbesserung der Ausbeute konnte durch Überexpression der Proteine MoaA und MoaC erreicht werden, die den ersten Schritt der Moco-Synthese katalysieren. Dieses führte zu einer etwa 5000-fachen Anreicherung von Precursor Z im Vergleich zu Wildtyp-Zellen, bei einer durchschnittlichen Ausbeute von 40 µg je L *E*. *coli* Kultur und einer Endkonzentration von 30-140 µg/ml. Gereinigter Precursor Z zeigte das gleiche Absorptionsspektrum wie zuvor beschrieben^{B19} und konnte quantitativ zu Compound Z oxidiert werden (Fig. 5a, b). Unter physiologischen und aeroben Bedingungen bei pH 6,9 (2x PBS Puffer) wurde eine 22 µM Lösung von Precursor Z innerhalb von 5 h vollständig zu Compound Z oxidiert^{B19}. Die Halbwertszeit von Precursor Z bei pH 6,9 konnte mit 62±12 min bestimmt werden (Fig. 5c).

### Wiederherstellung der MPT-Biosynthese in vitro

Die biologische Aktivität des gereinigten Precursor Z konnte durch eine quantitative *in vitro* Umwandlung zu MPT unter Verwendung gereinigter *E. coli* MPT-Synthase nachgewiesen werden^{B26}. Bei einem zweifachen Überschuss von MPT-Synthase waren wir in der Lage, Precursor Z nahezu komplett in MPT umzuwandeln, wie durch HPLC FormA Analyse bestimmt wurde (Daten nicht gezeigt). Um die Menge an Precursor Z zu ermitteln, die zu einer erfolgreichen Wiederherstellung der Moco-Biosynthese in *MOCS1*-defizienten Mäusen benötigt wird, haben wir eine *in vitro* Rekonstitution mit verschiedenen Mengen an Precursor Z in Protein-Rohextrakt aus der Leber von fünf Tage alten MOCS1-defizienten Mäusen durchgeführt (Fig. 6a). Beim Einsatz von 134 ng Precursor Z / mg Protein konnte eine nahezu komplette Wiederherstellung der Moco-Biosynthese erreicht werden, während 27 ng Recursor Z zu einer Rekonstitution von etwa 50 % führten. Der Protein-Gesamtgehalt des für dieses Experiment verwendeten Leberextraktes betrug 6 mg (erhalten aus 50 mg Gesamt-Lebergewebe), was eine gesamt erforderliche Menge Precursor Z von 0,8 µg je Tier zur vollständigen Wiederherstellung der MPT-Synthese unter *in vitro* Bedingungen erforderlich macht. Dementsprechend würden für ein erwachsenes Tier mit einem Köpergewicht von 20 g und einer ungefähren Masse der Leber von 1 g 16 µg Precursor Z zur vollständigen Wiederherstellung benötigt werden, jedoch nur 3 µg für eine Aktivierung von 50 %. Da die Produktionsmöglichkeiten für Precursor Z begrenzt waren, haben wir in den folgenden Experimenten nicht versucht, eine komplette Wiederherstellung zu erreichen, sondern nur die minimal notwendigen Dosierungen verwendet. Obwohl das *in vitro* Titrationsexperiment keinen Aufschluss darüber gibt, welcher Anteil des Moco auf die Apo-Sulfit-Oxidase übertragen wird, bezieht sich die ermittelte Menge an Precursor Z auf die maximale Kapazität der MPT-Produktion in der Leber *MOCS1*-defizienter Mäuse.

### Heilung des lethalen Phänotyps in Moco-defizienten Mäusen

Es konnte sicher nachgewiesen werden, dass ausschliesslich der Verlust der Sulfit-Oxidase Aktivität für die schweren neurologischen Schäden verantwortlich ist, die als Folge von Moco-Defizienz auftreten^{B11}. Die Halbwertszeit von Sulfit-Oxidase in Rattenleber wurde auf 3-4 Tage bestimmt^{B27}. Auf dieser Grundlage und anhand der oben aufgeführten Berechnungen haben wir Precursor Z in die Leber injiziert, wie in Tabelle 2 zusammengefasst. Während nicht behandelte homozygote Tiere im Vergleich zu ihren Artgenossen desselben Wurfs deutlich langsamer an Gewicht zunahmen und sehr früh starben^{B25}, war es optisch ohne Bestimmung des Genotyps unmöglich, behandelte homozygote Tiere in einem Wurf zu identifizieren. Bezüglich des Gewichts, des Verhaltens oder anderer phänotypischer Merkmale konnten bis zur Entwöhnung keine Unterschiede zwischen Precursor Z-behandelten *MOCS1*-defizienten Mäusen und Wildtyp- bzw. heterozygoten Individuen eines Wurfs festgestellt werden.

| **Tabelle 2 Lebensspanne von *MOCS*1 -/- Mäusen bei unterschiedlichen Behandlungen** | | |
|---|---|---|
| **Behandlung^{a}** | **n** | **Durchschnittliche Lebensspanne (Tage)** |
| Unbehandelt^{b} | 28 | 7,5 |
| 0,2 *µ*g jeden zweiten Tag | 11 | 17,7 |
| 1 *µ*g jeden dritten Tag | 5 | 25,2 |
| 1 *µ*g (vor Entwöhnung^{c}) und 2 µg (nach Entwöhnung) jeden dritten Tag | 30 | 32,9 |
| 2 µg Precursor Z jeden zweiten Tag | 15 | 47,4 |
| 2 *µ*g (vor Entwöhnung) und 4 µg (nach Entwöhnung) jeden dritten Tag | 18 | 91,8 |

| | | |
|---|---|---|
| ^{a}Precursor Z wurde durch die Haut in die Leber injiziert. ^{b}Daten von Quelle B16. ^{c}Entwöhnung fand statt zwischen Tag 20 und 30 nach der Geburt. | | |

Wie aus Tabelle 2 hervorgeht, gibt es eine starke Korrelation zwischen der pro Woche injizierten Menge an Precursor Z und der durchschnittlichen Lebensspanne der *MOCS1*-defizienten Mäuse. Die niedrigste Dosierung betrug 0,2 µg Precursor Z, verabreicht jeden zweiten Tag. Bei dieser Behandlung entwickelten sich 11 *MOCS1*^{*-*/*-*} Mäuse aus drei Würfen normal und nahmen entsprechend den Artgenossen an Gewicht zu. Obwohl keine Unterschiede im Verhalten festgestellt werden konnten, wurden alle 11 homozygoten Tiere zwischen den Tagen 14 und 19 gezielt von ihren Eltern getötet. Bei einer Gabe von 1-2 µg jeden dritten Tag erreichten die Mäuse ein höheres Alter und wurden bis Tag 20-30 gesäugt, danach erschienen die *MOCS1*^{*-*/*-*} Tiere jedoch langsamer in ihren Fluchtreaktionen und in der Wahrnehmung ihrer Umgebung als die entsprechenden Kontrolltiere. Dieser Unterschied verschwand um den Tag 40 herum, nachfolgend waren alle *MOCS1*^{*-*/*-*} Tiere so agil und lebhaft wie die Kontrollindividuen. Etwa die Hälfte der behandelten Tiere starb zwischen Tag 20 und 40. Als erfolgreichste Behandlung erwies sich die Injektion von 2-4 µg Precursor Z jeden dritten Tag. Während dieser Behandlung starb kein Tier zwischen den Tagen 40-70, einer Zeitspanne, in der Männchen und Weibchen das Erwachsenenstadium und die Fortpflanzungsfähigkeit erreichten. Zehn Paarungen dieser behandelten Tiere untereinander und vier weitere mit *MOCS1* Heterozygoten (zwei Paarungen mit homozygoten Männchen und zwei mit homozygoten Weibchen) ergaben normal ausgetragenen, gesunden Nachwuchs, der im Falle des *MOCS1*^{*-*/*-*} Phänotyps wiederum mit Precursor Z behandelt werden musste.

### Verhinderung von neurologischem Schaden

Die allgemeine Aktivität der behandelten Tiere (2-4 µg Precursor Z jeden dritten Tag) wurde im "Open Field" Test ermittelt, wobei die untersuchten Parameter (Bewegung, Lernen und Sozialverhalten) keine signifikanten Unterschiede zwischen Wildtyp (n=6) und homozygoten Tieren (n=8) eines Wurfs ergaben. Vermeidung von Licht wurde in der Hell-Dunkel-Box untersucht, wobei die Tiere in den hellen Bereich gesetzt wurden und die Zeit bestimmt wurde, bis die Tiere den dunklen Bereich aufsuchten. Auch dieser Test ergab keine signifikanten Unterschiede zwischen Wildtyp und *MOCS1*-defizienten, Precursor Z behandelten Mäusen (Daten nicht gezeigt).

Einschränkungen der Bewegungen wurden für *MOCS1*-defiziente und Precursor Z behandelte Mäuse im Laufrad Test nicht festgestellt (10 und 20 Umdrehungen pro Minute, Daten nicht angegeben). Die Lernfähigkeit der *MOCS1*-defizienten Mäuse, die regelmäßig Precursor Z erhielten, wurde in einem in vier Sektoren unterteilten Wasserlabyrinth ermittelt, das in einem Sektor eine unsichtbare Plattform enthielt. Die Ergebnisse wurden mit Wildtyp-Kontrolltieren verglichen. Die Mäuse wurden der Reihe nach in alle vier Sektoren gebracht und es wurde die Zeit gemessen, bis die Tiere die Plattform fanden und betraten (Fig. 7). In der ersten Versuchsreihe fanden die behandelten "Knock-out"-Mäuse (n=6) die Plattform schneller als die Wildtyp-Kontrolltiere (n=6). In einer zweiten Versuchsreihe verringerte sich die durchschnittliche Schwimmzeit gleichermaßen auf 20-25 % des ersten Versuchs sowohl bei behandelten Knock-out"-Mäusen als auch bei Kontrolltieren. In einer dritten Versuchsreihe wurde die Plattform entfernt und die Zeit gemessen, die die Mäuse in dem "richtigen" Sektor verbrachten, der zuvor die Plattform enthalten hatte. Beide Gruppen verbrachten etwa 50 % der Zeit in dem korrekten Sektor.

### Auftreten des Defizienz-Phänotyps nach Absetzen der Behandlung

Um zu untersuchen, ob die Behandlung mit Precursor Z nach bestimmten Entwicklungsstadien eingestellt werden kann, stoppten wir die Injektion von Precursor Z bei homozygoten Mutanten an den Tagen 1 (n=3), 12 (n=4), 26-28 (n=3) und 64-68 (n=2) nach der Geburt. Eine einzige Injektion von Precursor Z an Tag 1 führte zum Tod nach 15-18 Tagen. Das Absetzen von Precursor Z an Tag 12 resultierte in verringerter Erkundung der Umgebung, beginnend an Tag 18 (d.h. 6 Tage nach der letzten Injektion), und dem Tod der Tiere 1-3 Tage später (d.h. 7-9 Tage nach der letzten Injektion). Im Alter von 26-28 Tagen, was in die zuvor beschriebene kritische Periode fällt, führte das Absetzen von Precursor Z zum Tod innerhalb von 4-7 Tagen. Absetzen nach 64-68 Tagen führte zu verringerter Erkundung der Umgebung nach 7 Tagen und zum Tod nach 9-10 Tagen. Um den Phänotyp erfolgreich zu beheben, musste die Versorgung mit Precursor Z innerhalb von 5 Tagen nach der Geburt aufgenommen werden. Eine Aufnahme der Behandlung nach 7 bzw. 10 Tagen führte zu keiner signifikanten Verbesserung des Phänotyps oder der Lebenserwartung (Daten nicht gezeigt).

### Wiederherstellung von Moco-Synthese und Moco-abhängigen Enzym-Aktivitäten in vivo

Bei homozygoten *MOCS1*^{-/-} Tieren, denen regelmäßig Precursor Z injiziert wurde und die hinsichtlich Gewicht, Aussehen und Verhalten normal erschienen, wurde die Behandlung eingestellt und die Tiere wurden nacheinander an Tag 0, 1, 4 und 7 nach der letzten Injektion getötet, um den MPT-Gehalt und die Mo-Enzymaktivitäten in der Leber zu ermitteln (Fig. 6b-d). Am Tag der Injektion wurde aufgrund der schnellen Umwandlung des verabreichten Precursor Z zu MPT der höchste MPT-Wert gemessen (16±1% des Wildtyps; Fig. 6b). Innerhalb der folgenden vier Tage fiel der MPT-Gehalt kontinuierlich auf unter 4 % des Wildtyp-Wertes.
Die Aktivität der Sulfit-Oxidase zeigte einen Anstieg von Tag 0 zu Tag 1 nach der letzten Injektion (Fig. 6c). Sowohl Sulfit-Oxidase- als auch Xanthin-Dehydrogenase-Aktivität erreichten einen relativen Wert (angegeben in % des Wildtyps), der höher war als der rekonstituierte MPT-Gehalt (Fig. 6d). Nach Tag 1 nahm die Xanthin-Dehydrogenase-Aktivität schnell ab. Morphologische Untersuchungen von Precursor Z-behandelten Knock-out Mäusen ergaben beidseitig abnormale Nieren ab Tag 20 nach der Geburt (Fig. 8a, b). Dieses korreliert mit den sehr hohen Werten von Xanthin sowie nicht messbaren Werten von Harnsäure im Urin (Daten nicht gezeigt). Neben den Nierenschäden wurden keine weiteren morphologischen Abnormitäten beobachtet. Untersucht wurden Gewebe aus Herz, Skelettmuskel, Lunge, Bauchspeicheldrüse, Schilddrüse, Milz und Leber (Daten nicht gezeigt) sowie Gehirn (Fig. 8c-d).

### Diskussion

Die Wiederherstellung von muriner Moco-Biosynthese durch bakteriellen Precursor Z *in vivo* und *in vitro* unterstützt die genetischen Hinweise auf einen einzigartigen und hoch konservierten Syntheseweg, der zum Moco und Moco-abhängigen Enzymaktivitäten führt¹². Wir beobachteten eine eindeutige Korrelation zwischen der regelmäßig verabreichten Menge an Precursor Z und der Lebensdauer von *MOCS1*-defizienten Mäusen (Tabelle 2). Die gezielte Tötung des betroffenen Nachwuchses durch die Eltern infolge der niedrigsten hier verabreichten Dosierung könnte entweder auf einen unterschiedlichen Geruch - möglicherweise verursacht durch erhöhte Werte an schwefelhaltigen Verbindungen - oder geringfügige Änderungen im Sozialverhalten hindeuten. Die hohe Sterblichkeit zwischen Tag 20-40 nach der Geburt, die nach Behandlung mit mittleren Mengen Precursor Z (1-2 µg) und Absetzen der Behandlung eintrat, könnte durch eine kritische Phase in der Gehirnentwicklung bedingt sein, während dieser die Tiere sehr empfindlich auf erhöhte Sulfit- bzw. verminderte Sulfatkonzentrationen reagieren. Bei einer hohen Dosierung nimmt die Aktivität der Sulfit-Oxidase innerhalb des ersten Tages der Injektionen von Precursor Z in die Leber zu, was darauf hindeutet, dass (i) das Moco aufnehmende Apoenzym weniger stabil ist als das Holoenzym, (ii) die verfügbaren Apoenzym-Moleküle schnell mit Moco saturiert werden und (iii) Apoenzym neu synthetisiert werden muss, um sämtlichen verfügbaren Moco aufzunehmen. Verglichen mit dem Wildtyp-Wert überschreitet sowohl die Aktivität der Sulfit-Oxidase als auch die der Xanthin-Dehydrogenase den Prozentsatz an wiederhergestelltem MPT (Fig. 6). Diese Erkenntnis lässt auf einen sehr effizienten Einbau von verfügbarem Moco in die Apoenzyme sowie auf eine Fraktion von Moco im Wildtyp, der nicht an Mo-Enzyme gebunden ist, schließen. Darüber hinaus stimmt diese Erkenntnis mit früheren Beobachtungen bei Gephyrin-defizienten Zelllinien überein, wo geringste Mengen an wiederhergestelltem Moco zu einer überproportional hohen Aktivität der Sulfit-Oxidase führten^{B22}.

Soweit wir wissen, ist die Stabilität der Xanthin-Dehydrogenase *in vivo* bisher nicht bestimmt worden, jedoch wurde die Halbwertszeit der zugehörigen mRNA auf 12-16 Stunden geschätzt^{B28}, was der Größenordnung der Halbwertszeit der Enzymaktivität entspricht, wie sie anhand der Unterschiede zwischen Tag 1 und 4 nach Precursor Z-Injektion geschätzt wurde (Fig. 6d). Die Aktivität der Sulfit-Oxidase entspricht nach Erreichen des Maximums an Tag 1 der veröffentlichten Halbwertszeit von 3-4 Tagen^{B27} (Fig. 6c). Möglicherweise ist die durchschnittliche Aktivität der Xanthin-Dehydrogenase aufgrund der starken Fluktuationen niedriger als die Aktivität der Sulfit-Oxidase. Dieses ist der Hauptgrund für die Entstehung von Xanthin-Steinen bei klassischer humaner Xanthinurie (Typ 1) und letztendlich für ein Versagen der Nieren verantwortlich und scheint somit auch die Todesursache der älteren behandelten Tiere nach 70 Tagen zu sein. Humane Xanthinurie bleibt jedoch bis auf das gänzliche Fehlen von Xanthin-Dehydrogenase-Aktivität in den meisten Fällen ohne Symptome^{B29}, und selbst in Fällen mit Symptomen kommt es nur selten zu lebensbedrohlichen Zuständen. Daher muss in diesem Zusammenhang ein zusätzlicher Einfluss der Sulfit-Oxidase-Defizienz auf die Nierenfunktion in Betracht gezogen werden. So sollte die Behandlung humaner Moco-Defizienz primär auf die Wiederherstellung der Sulfit-Oxidase-Aktivität abzielen. Bis zum heutigen Tag wurde keine effektive Therapie für Patienten mit Moco-Defizienz beschrieben^{B11}. Für die seltene Typ C Defizienz, die durch Gephyrin-Mutationen verursacht wird, haben wir mittels Molybdat die Wiederherstellung in einer Fibroblasten-Kultur gezeigt, was bereits darauf hinwies, dass schon kleine Mengen von aktivem Moco für eine Wiederherstellung des normalen Phänotyps ausreichend sind^{B22}. Da zwei Drittel der bekannten Moco-Defizienz-Patienten zur Defizienz-Gruppe A gehören^{B10,B12} und alle Komponenten für eine Umwandlung von Precursor Z zu MPT bei diesen Patienten vorhanden sind^{B20,B24}, stellt gereinigter Precursor Z das erste potenziell effektive Heilmittel für die meisten Moco-Patienten dar.

Intravenöse Zuführung von Precursor Z ist eine attraktive Anwendungs-Option für humane Patienten. Dieses beinhaltet jedoch nicht nur eine neue Größenordnung der Precursor Z-Produktion, um dem etwa 1000-fach höheren Körpergewicht von Menschen gerecht zu werden, sondern auch eine starke Verdünnung, bevor der Wirkstoff die Leberzellen erreicht. Daher sollte bei Menschen die Verwendung eines permanenten Zugangssystems zur Leber in Betracht gezogen werden. Da die *MOCS* ^{-/-} Mäuse hinsichtlich der biochemischen Parameter dem Phänotyp humaner Patienten entsprechen, erscheint eine Behandlung der humanen Defizienz mit Precurrsor Z auf der Ebene von enzymatischer Wiederherstellung und Normalisierung des Stoffwechsels sehr vielversprechend.
Es wird sich erweisen müssen, ob eine verspätete Aufnahme der beschriebenen Therapie (was in den meisten Fällen aufgrund der benötigten Zeit zur klinischen Feststellung und Diagnose zu erwarten ist) noch dazu geeignet sein wird, einen neurologischen Schaden zu verhindern. Die beschriebene Precursor Z-Therapie bildet die Basis zur detaillierten Untersuchung des Fortschreitens bzw. Ausbleibens dieser Symptome, da wir nun auch in der Lage sind, durch Veränderung der Wirkstoff-Dosierung zu jeder Zeit Moco-Defizienz im tierischen Modell zu induzieren.
Tetrahydrobiopterin-Defizienz^{B30} ist ein ähnlicher Defekt in der Biosynthese von Tetrahydrobiopterin, was zum Verlust von NO-Synthasen^{B32} und der Hydroxylasen aromatischer Aminosäuren führt^{B31}, die essentiell sind für die Synthese von Neurotransmittern (Dopamin und Serotonin). Die Möglichkeiten der chemischen Synthese erlaubten bereits 1979 die erste erfolgreiche Behandlung eines Patienten mit Tetrahydrobiopterin-Defizienz^{B33,B34}; was mittlerweile eine etablierte Therapie darstellt^{B30}. Daher legen die Ergebnisse unserer Arbeit nachdrücklich klinische Versuchsstudien zur Behandlung von Moco-Defizienzpatienten nahe, sobald ausreichende Mengen an gereinigtem Precursor Z produziert werden können.

### Literatur

1. Hille, R. (1996) Chem. Rev. 96, 2757-2816
2. Stiefel, E. I. (1996) Science 272, 1599-1600
3. Enemark, J. H., and Cosper, M. M. (2002) Met. Ions Biol. Syst. 39, 621-654.
4. Mendel, R. R., Schwarz, G. (1999) Crit. Rev. Plant Sci. 18, 33-69
5. Johnson, J. L., Hainline, B. E., and Rajagopalan, K. V. (1980) J. Biol. Chem. 255, 1783-1786
6. Schindelin, H., Kisker, C., and Rajagopalan, K. V. (2001) Adv. Protein Chem. 58, 47-94
7. Mendel, R. R., and Schwarz, G. (2002) Met. Ions Biol. Syst. 39, 317-368
8. Duran, M., Beemer, F. A., van de Heiden, C., Korteland, J., de Bree, P. K., Brink, M., Wadman, S. K., and Lombeck, I. (1978) J. Inherit. Metab. Dis. 1, 175-178
9. Reiss, J., and Johnson, J. L. (2003) Hum. Mutat. 21, 569-576.
10. Johnson, J. L., and Duran, M. (2001) in The metabolic and molecular bases of inherited disease (Scriver, C., Beaudet, A., Sly, W., and Valle, D., eds), 8 Ed., pp. 3163-3177, McGraw-Hill, New York.
11. Fischer, B., and Burgmayer, S. J. (2002) Met. Ions Biol. Syst. 39, 265-316.
12. Fischer, B., Enemark, J. H., and Basu, P. (1998) J. Inorg. Biochem. 72, 13-21.
13. Rajagopalan, K. V., and Johnson, J. L. (1992) J. Biol. Chem. 267, 10199-10202
14. Wuebbens, M. M., and Rajagopalan, K. V. (1993) J. Biol. Chem. 268, 13493-13498
15. Rieder, C., Eisenreich, W., O'Brien, J., Richter, G., Götze, E., Boyle, P., Blanchard, S., Bacher, A., and Simon, H. (1998) Eur. J. Biochem. 255, 24-36
16. Pitterle, D. M., and Rajagopalan, K. V. (1989) J. Bacteriol. 171, 3373-3378
17. Pitterle, D. M., Johnson, J. L., and Rajagopalan, K. V. (1993) J. Biol. Chem. 268, 13506-13509
18. Gutzke, G., Fischer, B., Mendel, R. R., and Schwarz, G. (2001) J. Biol. Chem. 276, 36268-36274.
19. Rudolph, M. J., Wuebbens, M. M., Rajagopalan, K. V., and Schindelin, H. (2001) Nat. Struct. Biol. 8, 42-46.
20. Kuper, J., Palmer, T., Mendel, R. R., and Schwarz, G. (2000) Proc. Natl. Acad. Sci. U S A 97, 6475-6480.
21. Nichols, J., and Rajagopalan, K. V. (2002) J. Biol. Chem. 277, 24995-25000.
22. Johnson, J. L., Bastian, N. R., and Rajagopalan, K. V. (1990) Proc. Natl. Acad. Sci. U. S. A. 87, 3190-3194
23. Rajagopalan, K. V. (1996) in Escherichia coli and Salmonella typhimurium (Neidhardt, F. C., ed), pp. 674-679, ASM Press, Washington DC
24. Romao, M. J., Archer, M., Moura, I., Moura, J. J., Le-Gall, J., Engh, R., Schneider, M., Hof, P., and Huber, R. (1995) Science 270, 1170-1176
25. Chan, M. K., Mukund, S., Kletzin, A., Adams, M. W. W., and Rees, D. C. (1995) Science 267, 1463-1469
26. Wuebbens, M. M., and Rajagopalan, K. V. (2003) J. Biol. Chem. 278, 14523-14532.
27. Doyle, W. A., Burke, J. F., Chovnick, A., Dutton, F. L., Whittle, J. R., and Bray, R. C. (1996) Eur. J. Biochem. 239, 782-795.
28. Johnson, J. L., Wuebbens, M. M., and Rajagopalan, K. V. (1989) J. Biol. Chem. 264, 13440-13447
29. Reiss, J., Kleinhofs, A., and Klingmuller, W. (1987) Mol. Gen. Genet. 206, 352-355
30. Rivers, S. L., McNairn, E., Blasco, F., Giordano, G., and Boxer, D. H. (1993) Mol. Microbiol. 8, 1071-1081
31. Leimkuhler, S., and Rajagopalan, K. V. (2001) J. Biol. Chem. 276, 22024-22031.
32. Schäfer, A., Paul, H., Fischer, B., Hesse, M., and Viscontini, M. (1995) Helv. Chim. Acta 78, 1763-1776
33. Soyka, R., Pfleiderer, W., and Prewo, R. (1990) Helv. Chim. Acta 73, 808-826
34. Schindelin, H., Kisker, C., Hilton, J., Rajagopalan, K. V., and Rees, D. C. (1996) Science 272, 1615-1621
35. Kisker, C., Schindelin, H., Pacheco, A., Wehbi, W. A., Garrett, R. M., Rajagopalan, K. V., Enemark, J. H., and Rees, D. C. (1997) Cell 91, 973-983
36. Pfleiderer, W. (1996) in Comprehensive Heterocyclic Chemistry (Katritzky, A. R., Reese, C. W., and Scriven, E. F., eds) Vol. 7, pp. 679, Pergamon, Oxford
37. Bracher, A., Eisenreich, W., Schramek, N., Ritz, H., Gotze, E., Herrmann, A., Gutlich, M., and Bacher, A. (1998) J. Biol. Chem. 273, 28132-28141.
38. Irby, R. B., and Adair, W. L., Jr. (1994) J. Biol. Chem. 269, 23981-23987
39. Wuebbens, M. M., and Rajagopalan, K. V. (1995) J. Biol. Chem. 270, 1082-1087
40. Hänzelmann, P., Schwarz, G., and Mendel, R. R. (2002) J. Biol. Chem. 277, 18303-18312

B1. Rajagopalan, K.V. & Johnson, J.L. The pterin molybdenum cofactors. J. Biol. Chem. 267, 10199-10202 (1992).
B2. Wahl, R.C., Hageman, R.V. & Rajagopalan, K.V. The relationship of Mo, molybdopterin, and the cyanolyzable sulfur in the Mo cofactor. Arch. Biochem. Biophys. 230, 264-273 (1984).
B3. Reiss, J. et al. Mutations in a polycistronic nuclear gene associated with molybdenum cofactor deficiency. Nat. Genet. 20, 51-53 (1998).
B4. Stallmeyer, B., Drugeon, G., Reises, J., Haenni, A.L. & Mendel, R.R. Human molybdopterin synthase gene: identification of a bicistronic transcript with overlapping reading frames. Am. J. Hum. Genet. 64, 698-705 (1999).
B5. Stallmeyer, B. et al. The neurotransmitter receptoranchoring protein gephyrin reconstitutes molybdenum cofactor biosynthesis in bacteria, plants, and mammalian cells. Proc. Natl. Acad. Sci. U. S. A. 96, 1333-1338 (1999).
B6. Mendel, R.R. & Schwarz, G. Biosynthesis and molecular biology of the molybdenum cofactor (Moco). Met. Ions Biol. Syst. 39, 317-368. (2002).
B7. Rajagopalan, K.V. Biosynthesis of the molybdenum cofactor. in Escherichia coli and Salmonella typhimurium (ed. Neidhardt, F.C.) 674-679 (ASM Press, Washington DC, 1996).
B8. Duran, M. et al. Combined deficiency of xanthine oxidase and sulphite oxidase: a defect of molybdenum metabolism or transport? J. Inherit. Metab. Dis. 1, 175-178 (1978).
B9. Johnson, J.L. et al. Inborn errors of molybdenum metabolism: combined deficiencies of sulfite oxidase and xanthine dehydrogenase in a patient lacking the molybdenum cofactor. Proc. Natl. Acad. Sci. U. S. A. 77, 3715-3719. (1980).
B10. Reiss, J. Genetics of molybdenum cofactor deficiency. Hum. Genet. 106, 157-163. (2000).
B11. Johnson, J.L. & Duran, M. Molybdenum cofactor deficiency and isolated sulfite oxidase deficiency. in The metabolic and molecular bases of inherited disease (eds. Scriver, C., Beaudet, A., Sly, W. & Valle, D.) 3163-3177 (McGraw-Hill, New York, 2001).
B12. Reiss, J. & Johnson, J.L. Mutations in the molybdenum cofactor biosynthetic genes MOCS1, MOCS2, and GEPH. Hum. Mutat. 21, 569-576. (2003).
B13. Johnson, J.L. et al. Molybdopterin synthase mutations in a mild case of molybdenum cofactor deficiency. Am. J. Med. Genet. 104, 169-173. (2001).
B14. Johnson, J.L., Rajagopalan, K.V. & Wadman, S.K. Human molybdenum cofactor deficiency. Adv. Exp. Med. Biol. 338, 373-378 (1993).
B15. Hoff, T., Schnorr, K.M., Meyer, C. & Caboche, M. Isolation of two Arabidopsis cDNAs involved in early steps of molybdenum cofactor biosynthesis by functional complementation of Escherichia coli mutants. J. Biol. Chem. 270, 6100-6107 (1995).
B16. Gray, T.A. & Nicholls, R.D. Diverse splicing mechanisms fuse the evolutionarily conserved bicistronic MOCS1A and MOCS1B open reading frames. RNA 6, 928-936 (2000).
B17. Hänzelmann, P., Schwarz, G. & Mendel, R.R. Functionality of alternative splice forms of the first enzymes involved in human molybdenum cofactor biosynthesis. J. Biol. Chem. 277, 18303-18312 (2002).
B18. Gross-Hardt, S. & Reiss, J. The bicistronic MOCS1 gene has alternative start codons on two mutually exclusive exons. Mol. Genet. Metab. 76, 340-343 (2002).
B19. Wuebbens, M.M. & Rajagopalan, K.V. Structural characterization of a molybdopterin precursor. J. Biol. Chem. 268, 13493-13498 (1993).
B20. Johnson, J.L., Wuebbens, M.M., Mandell, R. & Shih, V.E. Molybdenum cofactor biosynthesis in humans. Identification of two complementation groups of cofactor-deficient patients and preliminary characterization of a diffusible molybdopterin precursor. J. Clin. Invest. 83, 897-903 (1989).
B21. Reiss, J. et al. Human molybdopterin synthase gene: genomic structure and mutations in molybdenum cofactor deficiency type B. Am. J. Hum. Genet. 64, 706-711 (1999).
B22. Reiss, J. et al. A mutation in the gene for the neurotransmitter receptor-clustering protein gephyrin causes a novel form of molybdenum cofactor deficiency. Am. J. Hum. Genet. 68, 208-213. (2001).
B23. Johnson, J.L., Wuebbens, M.M. & Rajagopalan, K.V. The structure of a molybdopterin precursor. Characterization of a stable, oxidized derivative. J. Biol. Chem. 264, 13440-13447 (1989).
B24. Johnson, J.L. & Rajagopalan, K.V. Molybdopterin biosynthesis in man. Properties of the converting factor in liver tissue from a molybdenum cofactor deficient patient. Adv. Exp. Med. Biol. 338, 379-382 (1993).
B25. Lee, H.-J. et al. Molybdenum cofactor-deficient mice resemble the phenotype of human patients. Hum. Mol. Gen. 11, 3309-3317 (2002).
B26. Gutzke, G., Fischer, B., Mendel, R.R. & Schwarz, G. Thiocarboxylation of molybdopterin synthase provides evidence for the mechanism of dithiolene formation in metal-binding pterins. J. Biol. Chem. 276, 36268-36274. (2001).
B27. Ono, H. & Ito, A. Transport of the precursor for sulfite oxidase into intermembrane space of liver mitochondria: characterization of import and processing activities. J. Biochem. (Tokyo) 95, 345-352. (1984).
B28. Falciani, F., Ghezzi, P., Terao, M., Cazzaniga, G. & Garattini, E. Interferons induce xanthine dehydrogenase gene expression in L929 cells. Biochem. J. 285, 1001-1008. (1992).
B29. Simmonds, H.A., Reiter, S. & Nishino, T. Hereditary xanthinuria. in The metabolic and molecular bases of inherited disease (eds. Scriver, C.R., Beaudet, A.L., Sly, W.S. & Valle, D.) 1781-1797 (McGraw-Hill, New York, 1995).
B30. Blau, N., Thöny, B., Cotton, R.G.H. & Hyland, K. Disorders of Tetrahydrobiopterin and Related Biogenic Amines. in The metabolic and molecular bases of inherited disease (eds. Scriver, C., Beaudet, A., Sly, W. & Valle, D.) 1725-1776 (McGraw-Hill, New York, 2001).
B31. Fitzpatrick, P.F. Tetrahydropterin-dependent amino acid hydroxylases. Annu. Rev. Biochem. 68, 355-381. (1999). B32. Alderton, W.K., Cooper, C.E. & Knowles, R.G. Nitric oxide synthases: structure, function and inhibition. Biochem. J. 357, 593-615. (2001).
B33. Schircks, B., Bieri, J.H. & Viscontini, M. [Preparation and characterisation of pure 5,6,7,8-tetrahydro-L-neopterine and 5,6,7,8-tetrahydro-D-monapterine (author's transl)]. Helv. Chim. Acta 59, 248-252. (1976).
B34. Niederwieser, A., Curtius, H.C., Viscontini, M., Schaub, J. & Schmidt, H. Phenylketonuria variants. Lancet 1, 550. (1979).
B35. Rivers, S.L., McNairn, E., Blasco, F., Giordano, G. & Boxer, D.H. Molecular genetic analysis of the moa operon of Escherichia coli K-12 required for molybdenum cofactor biosynthesis. Mol. Microbiol. 8, 1071-1081 (1993).
B36. Reiss, J., Kleinhofs, A. & Klingmuller, W. Cloning of seven differently complementing DNA fragments with chl functions from Escherichia coli K12. Mol. Gen. Genet. 206, 352-355 (1987).
B37. Leimkuhler, S. & Rajagopalan, K.V. A sulfurtransferase is required in the transfer of cysteine sulfur in the in vitro synthesis of molybdopterin from precursor Z in Escherichia coli. J. Biol. Chem. 276, 22024-22031. (2001).
B38. Nason, A. et al. Invitro formation of assimilatory reduced nicotinamide adenine dinucleotide phosphate: nitrate reductase from a Neurospora mutant and a component of molybdenum-enzymes. Proc. Natl. Acad. Sci .U S A 68, 3242-3246 (1971).
B39. Morris, R. Developments of a water-maze procedure for studying spatial learning in the rat. J. Neurosci. Methods 11, 47-60. (1984).
B40. Schwarz, G., Boxer, D.H. & Mendel, R.R. Molybdenum cofactor biosynthesis. The plant protein Cnxl binds molybdopterin with high affinity. J. Biol. Chem. 272, 26811-26814 (1997).
B41. Johnson, J.L. et al. Prenatal diagnosis of molybdenum cofactor deficiency by assay of sulphite oxidase activity in chorionic villus samples. J. Inherit. Metab. Dis. 14, 932-937 (1991).
B42. Mendel, R.R. & Müller, A.J. A common genetic determinant of xanthine dehydrogenase and nitrate reductase in Nicotiana tabacum. Biochem. Physiol. Pflanzen 170, 538-541 (1976).
B43. Koshiba, T., Saito, E., Ono, N., Yamamoto, N. & Sato, M. Purification and properties of flavin- and molybdenum containing aldehyde oxidase from coleoptyles of maize. Plant Physiol. 110, 781-789 (1996).

### Folgende Abkürzungen wurden verwendet:

1D, ein-dimensional; 2D, zwei-dimensional; COSY, Korrelations-Spektroskopie; ESI, Elektrospray-Ionisation; Moco, Molybdäncofaktor; MS, Massenspektrometrie; MPT, Molybdopterin oder Metall-bindendes Pyranopterin-En-Dithiolat; Mo, Molybdän; NMR Nukleäre Magnetische Resonanz; W, Wolfram

## Patentansprüche

1. Verwendung von Precursor Z zur Herstellung eines Mittels zur Therapie humaner Molybdäncofaktor-Defizienz, die direkt oder indirekt auf eine veränderte Molybdäncofaktor-Synthese zurückzuführen ist, **dadurch gekennzeichnet, dass** als wesentlicher Bestandteil dieses Mittels Precursor Z verwendet wird.

2. Verwendung nach Anspruch 1, **dadurch gekennzeichnet, dass** der Precursor Z C₁₀H₁₄N₅O₈P als Molekülformel aufweist, ein Pyranopterin darstellt und ein geminales Diol in der Seitenkette trägt.

3. Verwendung nach Anspruch 1, **dadurch gekennzeichnet, dass** der Precursor Z C₁₀H₁₄N₅O₈P als Molekülformel aufweist, ein Pyranopterin darstellt und ein geminales Diol in der C1'-Position trägt.

4. Verwendung nach Anspruch 1, **dadurch gekennzeichnet, dass** der Precursor Z 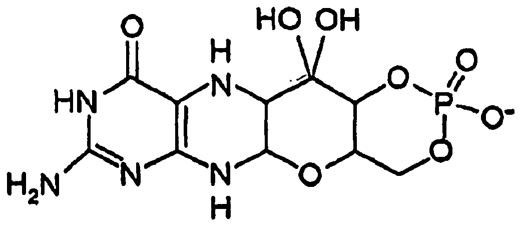 als Struktur formel aufweist, ein Pyranopterin darstellt und ein geminales Diol in der C1'-Position trägt.

## Claims

1. Use of precursor Z for the preparation of an active for the therapy of human molybdenum cofactor deficiency which can be directly or indirectly attributed to an altered molybdenum cofactor synthesis, **characterised in that** precursor Z is used as an essential component of said active.

2. Use according to claim 1, **characterised in that** the precursor Z has the molecular formula C₁₀H₁₄N₅O₈P, is a pyranopterin und carries a geminal diol in the side chain.

3. Use according to claim 1, **characterised in that** the precursor Z has the molecular formula C₁₀H₁₄N₅O₈P, is a pyranopterin und carries a geminal diol at position C1'.

4. Use according to claim 1, **characterised in that** the precursor Z has the structural formula 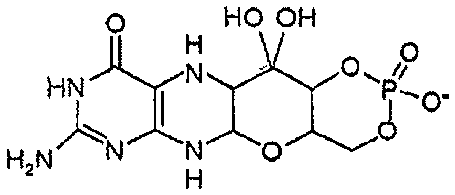 is a pyranopterin und carries a geminal diol at position C1'.

## Revendications

1. Utilisation du précurseur Z pour la fabrication d'un agent pour le traitement de la déficience en cofacteur à molybdène humain qui est directement ou indirectement imputable à une synthèse modifiée du cofacteur à molybdène, **caractérisée en ce que** le précurseur Z est utilisé comme composant essentiel de cet agent.

2. Utilisation selon la revendication 1, **caractérisée en ce que** le précurseur Z présente la formule moléculaire C₁₀H₁₄N₅O₈P, comprend une pyranoptérine et porte un diol géminé dans la chaîne latérale.

3. Utilisation selon la revendication 1, **caractérisée en ce que** le précurseur Z présente la formule moléculaire C₁₀H₁₄N₅O₈P, comprend une pyranoptérine et porte un diol géminé en position C1'.

4. Utilisation selon la revendication 1, **caractérisée en ce que** le précurseur Z a la formule structurale 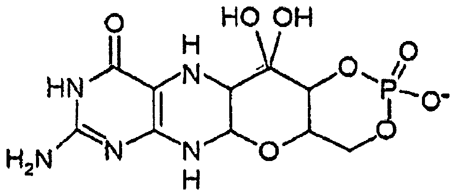 comprend une pyranoptérine et porte un diol géminé en position C1'.
